# EUROPEAN PATENT APPLICATION

(11) **EP 2 036 548 A1**
(43) Date of publication of application: **18.03.2009**
(21) Application number: 08169750.0
(22) Date of filing: 21.09.2004
(51) Int. Cl.: A61K 31/00, A61K 31/56, A61K 31/185, A61K 31/215, A61P 19/00, A61P 19/10, A61K 9/00

(54) **Prevention and treatment of inflammation-induced and/or immune-mediated bone loss**

(30) Priority: 22.09.2003 US 504717 P
(62) Divisional of application: 04765458.7
(71) Applicant: onepharm Research & Development GmbH, 1210 Wien (AT)
(72) Inventor: Wilckens, Thomas, 80796, München (DE); Volkmann, Ariane, 86 911, Diessen (DE)
(74) Representative: Redl, Gerda

(57) **Abstract**

The present invention relates to the use of an 11-β-HSD-type 1 and/or type 2 inhibitor for the manufacture of a pharmaceutical agent for the prevention and/or treatment of inflammation-induced and/or immune-mediated loss of bone and/or cartilage.

## Description

The present invention relates to the use of an 11-β-HSD-type 1 and/or type 2 inhibitor or a pharmaceutically acceptable salt thereof for the manufacture of a pharmaceutical agent for the prevention and/or treatment of inflammation-induced and/or immune-mediated loss of bone and/or cartilage.

Morphogenesis and remodelling of bone entail the synthesis of bone matrix by osteoblasts and the coordinate resorption of bone by osteoclasts. It has been estimated that about 10% of the total bone mass in humans is being remodelled each year. Osteoblasts and osteoclasts arise from distinct cell lineages and maturation processes, that is, osteoclasts arise from mesenchymal stem cells while osteoclasts differentiate from haematopoietic monocyte/macrophage precursors.

Imbalances between osteoclast and osteoblast activities can arise from a wide variety of hormonal changes or perturbations of inflammatory and growth factors, resulting in skeletal abnormalities characterized by decreased (osteoporosis) or increased (osteopetrosis) bone mass. In fact, in pathologic states associated with inflammation, "activated" cells (e.g., infiltrating leukocytes, synovial fibroblasts, and in particular T-cells) contribute other molecules that shift the balance between osteoblastic and osteoclastic activities resulting in debilitation bone erosion and/or osteoporosis.

Increased osteoclast activity is seen in many osteopenic disorders, including postmenopausal osteoporosis, Paget's disease, lytic bone metastases, or rheumatoid arthritis, leading to increased bone resorption and crippling bone damage. In addition, the T-cell features in diseased periodontal tissues can be compared with those in rheumatoid arthritis, wherein bone resorption often attributed to Th1-type T-cell involvement has also been demonstrated.

Various factors have been described including CSF1 (MCSF), IL1, TGFβ, TGFα, TNFα, TNFβ, IL6, vitamin 1,25-hihydroxyvitamin D3, IL11, calcitonin, PGE2, or parathyroid hormone (PTH) that affect osteoclastogenesis at distinct stages of development. However, genetic ablation experiments have shown that these factors are not essential for osteoclast development *in vivo*.

Because of the enormous social and economic impacts of bone loss and crippling to human welfare and the search to increase human life span without the "side effects" of old age, it was of paramount importance to identify essential factors involved in osteoclast development and bone remodelling.

The essential molecules have been recently identified to be the TNF-TNFR superfamily proteins RANKL, RANK, and OPG. The TNF family molecule RANKL (receptor activator of NFkB ligand; also known as osteoprotegerin ligand (RANKL); TNF related activation induced cytokine (TRANCE), osteoclast differentiation factor (ODF), and TNFSF11) and its receptor RANK (TNFRSF11A) are key regulators of bone remodeling and essential for the development and activation of osteoclasts. RANKL also regulates T cell/dendritic cell communications, dendritic cell survival,7 8 and lymph node organogenesis.

Moreover, production of RANKL by activated T cells directly controls osteoclastogenesis and bone remodeling and explains why autoimmune diseases, cancers, leukaemias, asthma, chronic viral infections, and periodontal disease result in systemic and local bone loss.

In particular, RANKL seems to be the pathogenetic principle that causes bone and cartilage destruction in arthritis. Inhibition of RANKL function via the natural decoy receptor osteoprotegerin (OPG, TNFRSF11B) prevents bone loss in postmenopausal osteoporosis and cancer metastases and completely blocks bone loss and crippling in various rodent models of arthritis. Intriguingly, RANKL and RANK play essential parts in the formation of a lactating mammary gland in pregnancy. This system provided a novel and unexpected molecular paradigm that links bone morphogenesis, T cell activation and the organization of lymphoid tissues, and mammary gland formation required for the survival of mammalian species.

Inhibition of inflammation-induced and or immune-mediated osteoclast activation by blocking the activation with small molecules might be the future treatment of choice to abolish osteoporosis, tooth loss, or crippling in arthritis as well as other inflammatory process associated with bone erosion or bone loss. The latter can be achieved by preventing T-cell activation as well as bone marrow infiltration with inflammatory cells, thus inhibiting contact interaction between T-cells and osteoclast precursors, or their respective receptors and ligands RANK and RANKL.

The following section outlines the scientific rational for preventing inflammation-induced osteoclast activation in specific diseases.

### Periodontal Disease:

Host inflammatory and immune responses to specific oral bacterial infections can result in periodontal disease, i.e., periodontitis (1). Human periodontitis is heterogeneous in etiology, but a common hallmark is alveolar bone destruction, one of the major clauses of tooth loss in human (2, 3). Interestingly, human periodontitis has recently been implicated in the increased risks of certain systemic disorders such as pre-term low birth weight, bacterial pneumonia, congestive heart diseases, and stroke (4-8), possibly due to an underlying inflammatory trait (9). About 10-12 subgingival microorganisms have been implicated in the pathogenesis of periodontitis, including *Porphyromonas gingivalis, Prevotella intermedia, Bacteroides forsythus*, and mixed spirochetes (10). In particular, *Actinobacillus actinomycetemcomitans*, a Gram-negative facultative capnophilic rod bacterium, has been identified as the etiological agent of localized juvenile periodontitis (LJP) and of some rapidly progressing and severe forms of periodontitis (10-13). The prevalence of LJP is about 1-4% among teens and young adults, and 10% among insulin-dependent diabetic patients (10). LJP is characterized by advanced alveolar bone destruction in a molar-incisor pattern that often leads to tooth mobility and loss, resulting in functional and aesthetic deficits. *A. actinomycetemcomitans* is able to invade the gingival epithelium (14) and releases several virulence factors such as cytotoxins, endotoxins, and a potent leukotoxin (15-17). *A. actinomyoetemcomitans* infection is usually accompanied by local and systemic antigen-specific immune responses (18-19). Earlier studies demonstrated altered CD4+/CD8+ T-cell ratios and autologous mixed lymphocyte reactions in LJP patients (20, 21) and the ability of T helper cells to home to periodontal tissues in rat and mouse models of periodontitis (22-24). Further, it was previously demonstrated that *A. actinomycetemcomitans* infection in NOD/SCID mice engrafted with human peripheral blood leukocytes (HuPBLs) leads to periodontal inflammation characterized by the infiltration of CD4+ T cells, CD8+ T cells, CD20+ B cells, and Mac1+ macrophages into the fibrous connective tissues adjacent to the periodontal pockets (24). These results suggested that T cells could modulate bacterium-induced periodontal inflammation and/or alveolar bone destruction. To investigate the precise mechanism or mechanisms that regulate periodontal immunity and alveolar bone destruction, HuPBLs from LJP patients were transplanted into NOD/SCID mice (which lack endogenous T and B cells), generating HuPBL-NOD/SCID mice (24). This study shows that oral challenge of these "humanized" mice with *A. actinomycetemcomitans* (designated Aa- HuPBL-NOD/SCID) leads to functional activation of the human CD4+ T cells in the periodontium and triggers local alveolar bone destruction. *In vitro* stimulation of CD4+ T cells from these mice with antigens from *A. actinomycetemcomitans* leads to the expression of osteoprotegerin ligand (OPGL, also known as TRANCE, ODF, and RANKL), a key mediator of osteoclastogenesis and osteoclast activation (25-31). Inhibition of OPG-L function via the decoy receptor osteoprotegerin (OPG) significantly reduces the alveolar bone destruction detected in Aa- HuPBL-NOD/SCID mice after bacterial inoculation, as well as the numbers of osteocfasts at the sites of local periodontal inflammation. These results identify for the first time a critical role for human CD4+ T cells reactive to oral microorganisms in periodontal disease. Moreover, *A. actinomycetemcomitans* -triggered induction of OPG-L expression on T cells and OPGL -mediated osteoclast activation and bone loss could provide one molecular explanation for the alveolar bone destruction observed in local periodontal infection. It has recently been stated, that the concept developed above can be translated to periodontal disease in general, since the latter pathology is always accompanied by an inflammatory process resulting in T-cell activation. Periodontal disease is the second most prevalent disease in the United States after heart disease. While it affects more than 50 million people at the moderate to severe level, only 15-20% receive treatment. Currently, more than $6 billion is spent annually to treat the disease in the U.S. Periodontal disease increases the susceptibility of oral tissue and bone to degradation by bacteria, creating pockets between the teeth and gums, thus making it a major cause of tooth loss. If left untreated, the implications of the disease extend well beyond the mouth. Studies have identified periodontal disease as a potential contributing factor to heart disease, diabetes, and low infant birth weight. The U.S. Surgeon General's Report 2000 further increased public visibility surrounding periodontal disease as a major healthcare issue. Current antimicrobial treatments cannot halt the ongoing bone destruction. Most likely a combination with small molecule preventing bone marrow infiltration with inflammatory cells and activation of T-cells will be an ideal treatment, which could be followed by a preventive strategy including the small molecule that blocks BM-infiltration.

### Rheumatoid arthritis:

Bone loss represents a major unsolved problem in rheumatoid arthritis (RA). The skeletal complications of RA consist of focal bone erosions and periarticular osteoporosis at sites of active inflammation, and generalized bone loss with reduced bone mass. New evidence indicates that osteoclasts are key mediators of all forms of bone loss in RA. TNF-α is one of the most potent osteoclastogenic cytokines produced in inflammation and is pivotal in the pathogenesis of RA. Production of tumor necrosis factor-α(TNF-α) and other proinflammatory cytokines in RA is largely CD4_ T-cell dependent and mostly a result of interferon-γ (IFN-γ) secretion. Synovial T cells contribute to synovitis by secreting IFN-γ and interleukin (IL)-17 as well as directly interacting with macrophages and fibroblasts through cell-to-cell contact mechanisms. Activated synovial T cells express both membrane- bound and soluble forms of receptor activator of NF-κB ligand (RANKL). In rheumatoid synovium, fibroblasts also provide an abundant source of RANKL. Furthermore, TNF-α and IL-1 target stromal-osteoblastic cells to increase IL-6, IL-11, and parathyroid hormone-related protein (PTHrP) production as well as expression of RANKL. Only in the presence of permissive levels of RANKL, TNF-α acts directly to stimulate osteoclast differentiation of macrophages and myeloid progenitor cells. In addition, TNF-α induces IL-1 release by synovial fibroblasts and macrophages, and IL-1, together with RANKL, is a major survival and activation signal for nascent osteoclasts. Consequently, TNF-α and IL-1, acting in concert with RANKL, can powerfully promote osteoclast recruitment, activation, and osteolysis in RA. The most convincing support for this hypothesis has come from *in vivo* studies of animal models. Protection of bone in the presence of continued inflammation in arthritic rats treated with osteoprotegerin (OPG) supports the concept that osteoclasts exclusively mediate bone loss, providing further evidence that OPG protects bone integrity by downregulating osteoclastogenesis and promoting osteoclast apoptosis.

The nexus between T-cell activation, TNF-α overproduction, and the RANKL/OPG/RANK ligand-receptor system points to a unifying paradigm for the entire spectrum of skeletal pathology in RA. Strategies that address osteoclastic bone resorption will represent an important new facet of therapy for RA.

### Osteoporosis in the aging population:

### A. Impact of cytokine changes with estrogen deficiency on osteoclastogenesis

There is progressive loss of bone tissue after natural or surgical menopause, leading to increased fractures within 15-20 yr from the cessation of ovarian function (271). Estrogen receptors (ER) have been detected in many cells that reside in bone tissue (272-278), suggesting that menopause may have direct consequences on cytokine secretion by cells located within the bone microenvironment. Bone marrow cells of the monocyte/macrophage lineage were believed to be the major source of the postmenopausal increases in TNF-α and IL-1 secretion in bone tissue (279). However, in the past few years it has been increasingly recognized that activated T cells are also an important source of increased TNF-α production in the bone marrow after menopause (195, 196, 209, 280-283). Proinflammatory cytokines are among the most powerful stimulants of bone resorption known. They directly and through the stimulation of other local factors intervene with every single step in osteoclastogenesis that determines the rate of bone resorption, from the proliferation and differentiation of the early osteoclast precursor cell to the resorption capacity and the lifespan of the mature osteoclast (9, 285- 301). The first step in osteoclastogenesis that determines the rate of bone resorption is the proliferation of osteoclast precursor cells. In fact, a major consequence of estrogen deficiency is the expansion of the pool of osteoclastic precursor cells in the bone marrow. Loss of ovarian function is permissive for the expression of the major cytokines that directly stimulate early osteoclast precursor proliferation, *i*.*e*., M-CSF, GM-CSF, and IL-6 (289, 301-307). Spontaneous increases in these cytokines may be further enhanced by the parallel increases in IL-1 and TNF-α with menopause, which are potent stimulators of M-CSF, GM-CSF (292, 298, 308-311), and IL-6 (64, 286, 306, 312-314). In summary, it can be stated that estrogen deficiency as observed after ovariectomy or in menopause is associated with an increased expression of mediatiors of inflammation. Furthermore, T cell deficiency effectively prevented bone loss in ovariectomized mice (199), clearly highlighting the RANK/RANKL pathway an essential mechanism contributing to enhanced osteoclast formation and bone loss. Of note, estrogen deficiency also appears to correlate with the incidence of several autoimmue deseases linking T-cell, B-cell activation with hormone status and bone physiology. As outlined above, bone loss with estrogen deficiency involves a large number of interrelated changes in estrogen-dependent regulatory factors (377). However, whereas in other proinflammatory conditions such as inflammatory arthritis, the deficiency in single proinflammatory cytokines does not fully prevent the inflammatory process (378), deficiency in several single cytokines is sufficient to completely block excessive bone resorption with estrogen deficiency. The redundancy of the function of most of these cytokines for osteoclast formation may compensate the lack of function of each of these components in situations apart from estrogen deficiency. The clear exceptions are M-CSF and the components of the RANKL/OPG/RANKsystem, whose activity is essential for osteoclast generation (199, 230, 317, 394-396). This evidence makes blockade of the T-cell interaction with osteoclast precursors a most attractive avenue for new therapeutic intervention in estrogen-induced bone loss; the latter being consider similar to inflammation-induced bone destruction.

### The cortisone/cortisol shuttle:

The interconversion of pharmacologically active cortisol and inactive cortisone is accomplished by two independent 11-β-hydroxysteroid dehydrogenases (11-β-HSD)3 that exhibit tissue-specific expression (1). Even though a third enzyme has been proposed, its existence has still to be demonstrated. In most intact cells, 11 β -HSD1 functions predominantly as a reductase, generating **active cortisol** from inactive cortisone and thereby enhancing activation of the glucocorticoid receptor. However, there is strong evidence, that the reaction direction might highly depend on the specific tissue type; thus in Leydig cells 11-β-HSD-1 may also function as a dehydrogenase. 11-β -HSD1 is broadly distributed among tissues, with predominant expression occurring in hepatic, adipose, gonadal, and central nervous system tissues. Mice with a targeted disruption of the 11- β -HSD1 gene are more resistant to hyperglycemia induced by stress or high-fat diet than their wildtype counterparts, consistent with the emerging notion that the activation of glucocorticoids by prereceptor metabolism may be central to the appearance of many sequelae of insulin resistance 2). 11 - β -HSD2, which is mainly expressed in the placenta and aldosterone target tissues such as the kidney and colon, acts almost exclusively as a dehydrogenase, thereby preventing the activation of mineralocorticoid receptor-sensitive genes by excess cortisol 1). 18-β-Glycyrrhetinic acid, an active component of licorice, is an inhibitor of 11-β-HSD1 as well as 11-β-HSD2, and licorice ingestion or administration of 18 β -glycyrrhetinic acid or its hemisuccinate derivative carbenoxolone results in hypertension and metabolic alkalosis due to inhibition of 11-β-HSD2 (3, 4) due to increased access to **active cortisol** to the mineralocorticoid receptors in the kidney. Patients with mutations in the gene encoding 11-β-HSD2 suffer from the syndrome of "apparent mineralocorticoid excess" entailing hypokalemia and severe hypertension (5). Similar symptoms also were recently described for the 11-β-HSD2 knockout mice (2). For several decades, synthetic glucocorticoids have found significant therapeutic use as anti-inflammatory agents in various diseases such as rheumatoid arthritis, allergic diseases, and bronchial asthma (6). Consistent with the pluripotent effects of glucocorticoids, the glucocorticoid receptor is widely distributed among peripheral tissues. In many instances, the tissue distribution of this receptor and that of 11-β-HSD1 are overlapping (1). Although glucocorticoids are commonly prescribed for their anti-inflammatory actions, to date relatively few studies address the involvement of 11-β-HSD in glucocorticoid-mediated immune functions. In one such study, the importance of pre-receptor metabolism by 11β-HSD enzymes in controlling inflammatory responses has been highlighted by demonstrating that pharmacological inhibition of 11β-HSD activity present in skin leads to an augmentation of the anti-inflammatory action of topically applied cortisol on contact hypersensitivity responses (7). The inhibitor applied alone displayed no effect. There it was proposed that blocking 11-β-HSD in the skin abrogated corticoid inactivation. Recently the expression of 11-β-HSD in a primary inflammatory effector cell, the monocyte/macrophage was investigated. These studies confirm the complete absence of both 11β-HSD1 and 11β- HSD2 in freshly isolated circulating human monocytes. However, 11β-reductase activity was induced during monocyte culture or after stimulation with the anti-inflammatory cytokines IL-4 and IL-13, strongly suggesting that it may play an important role in regulating the immune functions of these cells.

Since both isoenzymes were discovered in bone cells, it was further speculated that activation of cortisone by the dominant reductase activity of 11-β-HSD, e.g. exaggerated conversion to cortisol might be part of bone loss induced by glucocorticoids in general, including osteoporosis observed in rheumatoid arthritis. From this evidence one could speculate that blocking 11-β-HSD would result in enhanced bone loss.

Thus, while we had proposed that blocking 11-β-HSD would not only ameliorate arthritis by enabling tolerance induction due to increased local glucocorticoid concentrations, we were concerned that this treatment would increase bone destruction.

Surprisingly this is not the case. In fact, blockade of 11-β-HSD not only decreased inflammation, but also completely prevented bone marrow infiltration with inflammatory cells. Since it has been proposed that preosteoclasts are recruited from synovial as well as bone marrow monocytic cell lines, the prevention of infiltration must be considered the main effector pathway for the prevention of bone erosion in adjuvant arthritis and inflammation-induced bone destruction in general. The latter is further corroborated by the fact that the injection of 18-β-glycyrrhetinic acid needed to be in close proximity to draining lymph nodes in order to display clinical efficacy either alone or in conjunction with a peptide.

Therefore we propose that 11-β-HSD blockade increases local glucocorticoid concentrations in immune tissues which prevents the interaction between activated T-cells an osteoclast precursors and/or T-cell activation *per se*.

Given these findings it appears most unlikely that endogeous glucocorticoids contribute to bone loss during acute inflammation; the latter might possibly be the case under physiological non-inflammatory conditions. In fact, in rat adjuvant arthritis, an established model for the human disease, dexamethasone, a potent synthetic glucocorticoid in conjunction with a CD4+ depleting antibody, strongly protected rats from bone erosion. In addition, dexamethasone also enhanced anti-TNF-induced amelioration of synovial inflammation and bone erosion in rat models for rheumatoid arthritis. Thus increasing local glucocorticoid levels might have beneficial effects on bone and bone homeostasis during acute inflammation and/or during immune-mediated activation of bone destruction. Our findings clearly contraste the hypothesis recently put forward. In addition, 11-β-HSD expressed in osteoblasts is most unlikely to play a role in the present phenomenon, since activation of osteoclast is depending on the interaction with activated T-cells, and not osteoblast in bone marrow (Nature). This evidence further negates a functional role of osteoblastic 11-β-HSD in inflammation induced bone destruction. Based on our *in vivo* findings we investigated the gene expression of 11-β-HSD and biological activity in tissues relevant for immune function. For the first time we identified 11-β-HSD activity in dendritic cells and lymphoid cells (unpublished) in both, human and rat tissues. Most interestingly, taqman analysis indicates the presence of mRNA for more then one 11-β-HSDs. This evidence strongly suggest that 11-β-HSD might have a functional role in regulating immunity. In addition, the previously postulated type 3 enzyme might well be a homologue of the established type 2. It had earlier been proposed that differences might potentially exist within the known 11-β-HSD-2 enzyme (s) observed in placenta and kidney, since their cDNAs were similar but not identical.
Since 18β-glycyrrhetinic acid blocks both known as well as a putative third enzyme, it currently can not be definitely decided which enzyme is the most responsible for the beneficial effect of 11-β-HSD-blockade. The fact that inflammatory mediators such as cytokines can influence the balance between reductase and dehydrogenase activity either by altering the balance between the iso-enzymes or changing the reaction direction at the single enzyme level; necessitates the development of more selective inhibitors for identification of the relevant target.
Recent evidence establishes inflammation-induced and/or immune-mediated bone loss as an essential direct interaction between activated T-cells and osteoclast precursors. This crucial mechanism can be prevented by the use of 18-β-glycyrrhetinic acid and related compounds that modulate the cortisol/cortisone shuttle; i.e. 11-β-hydroxysteroid-dehydrogenase activity and/or expression as well as selective inhibitors useful for the modulation of 11-β-HSD.
It was an object of the present invention to provide a use of an 11-β-HSD-type 1 and/or type 2 inhibitor or a pharmaceutically acceptable salt thereof for the manufacture of a pharmaceutical agent for the prevention and/or treatment of inflammation-induced and/or immune-mediated loss of bone and/or cartilage. Using conventional drugs for the therapy of inflammations, it was observed that bone loss continues to go on, si nce osteoclast activation remains. It was found that bone loss can be prevented effectively by means of the 11-β -HSD inhbitors of the invention.

According to the invention, the 11-β-HSD-type 1 and/or type 2 inhibitors are preferably used for the prevention and/or treatment of bone and/or cartilage loss in a mammal, more preferably in a human.

In a preferred embodiment of the invention, the inflammation-induced and/or immune-mediated loss of bone and/or cartilage includes but is not limited to osteoporosis, postmenopausal osteoporosis, Paget's disease, lytic bone metastases, arthritis, juvenile chronic arthritis, adjuvant arthritis, infectious diseases, bone loss by cancer, bone loss by HIV, tooth loss, bone marrow inflammation, synovial inflammation, cartilage and/or bone erosion and/or proteoglycan damage.

In a more preferred embodiment of the invention, the immune-mediated loss of bone and/or cartilage includes osteoarthritis, rheumatoid arthritis and/or periodontitis.

Preferably, the 11-β-HSD-type 1 and/or type 2 inhibitors are selected from the group consisting of the following formulas:

| **Compound Name** | **Structure** |
|---|---|
| Formula 1 | 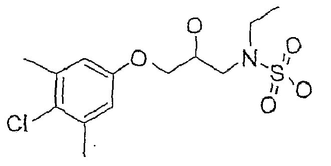 |
| Formula 2 | 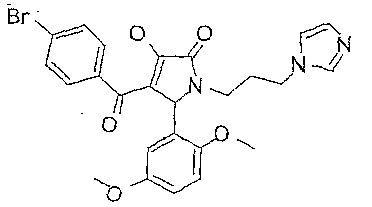 |
| Formula 3 | 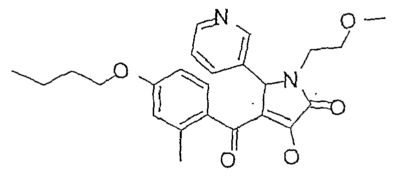 |
| Formula 4 | 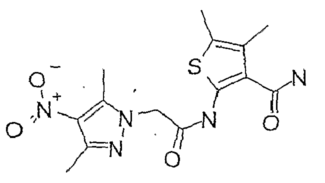 |
| Formula 5 | 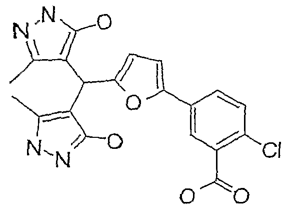 |
| Formula 6 | 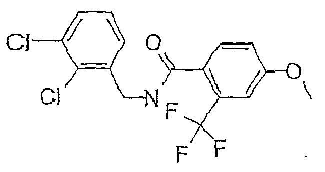 |
| Formula 7 | 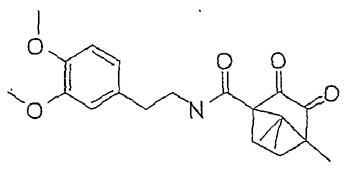 |
| Formula 8 | 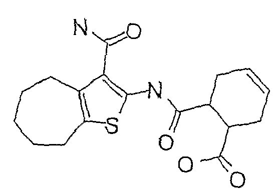 |
| Formula 9 | 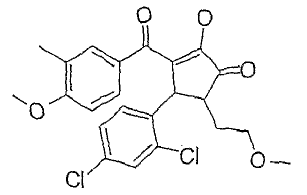 |
| Formula 10 | 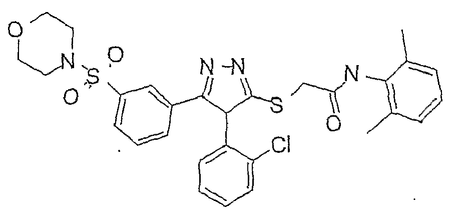 |
| Formula 11 | 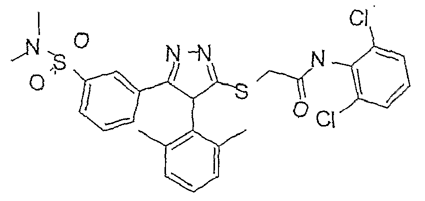 |
| Formula 12 | 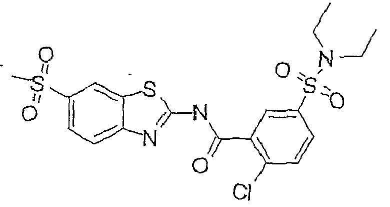 |
| Formula 13 | 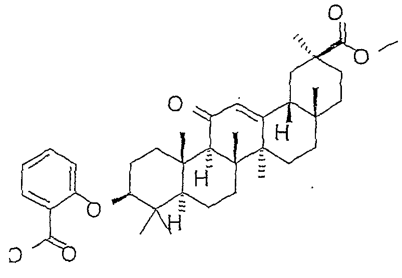 |
| Formula 14 | 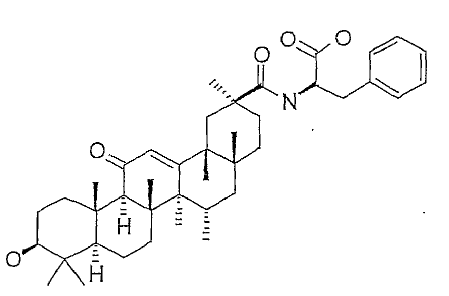 |
| Formula 15 | 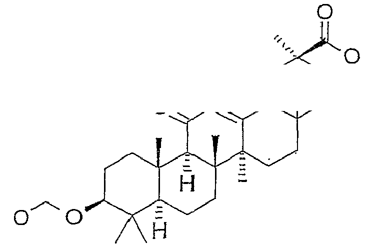 |
| Formula 16 | 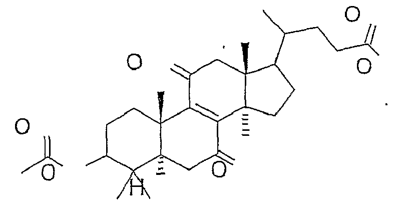 |
| Formula 17 | 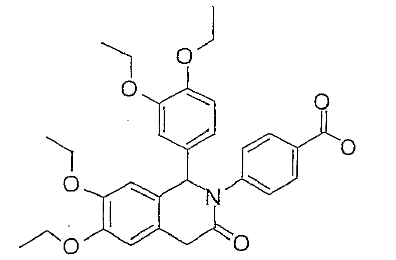 |
| Formula 18 | 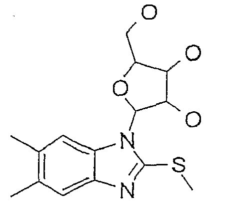 |
| Formula 19 | 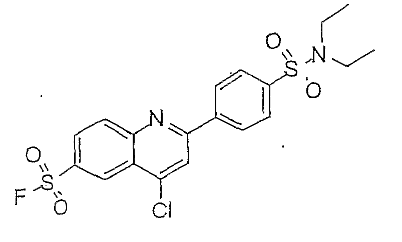 |
| Formula 20 | 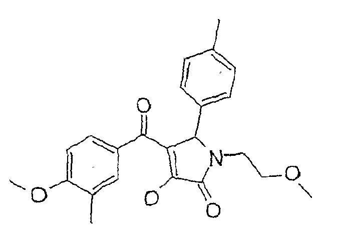 |
| Formula 21 | 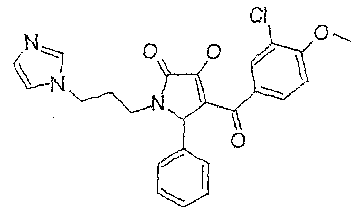 |
| Formula 22 | 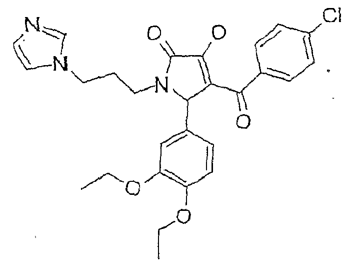 |
| Formula 23 | 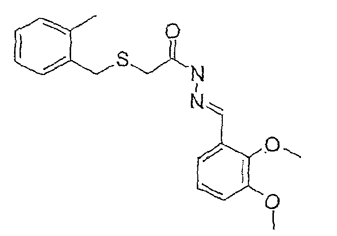 |
| Formula 24 | 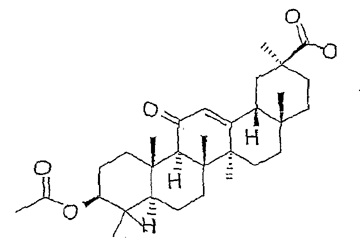 |
| Formula 25 | 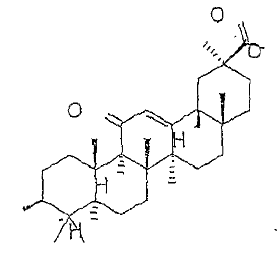 |
| Formula 26 | 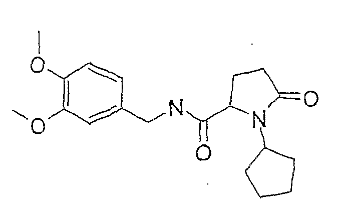 |
| Formula 27 | 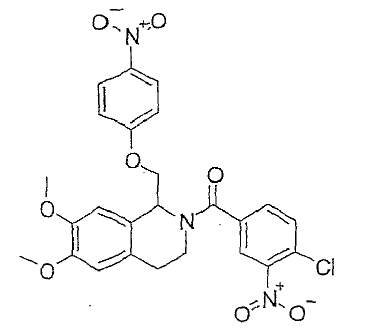 |
| Formula 28 | 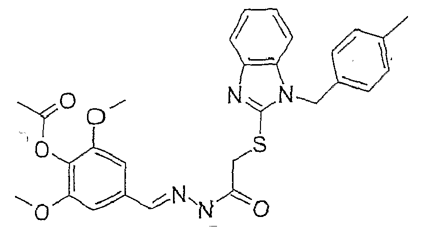 |
| Formula 29 | 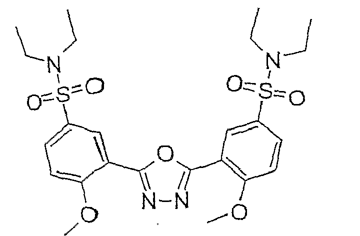 |
| Formula 30 | 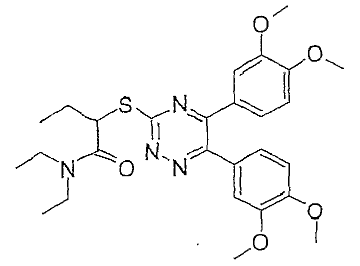 |
| Formula 31 | 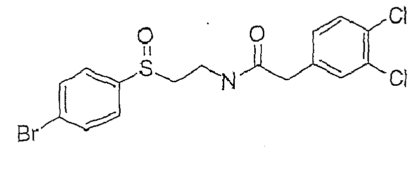 |

In another preferred embodiment, the 11-β-HSD-type and/or type 2 inhibitor has the structure of formula I: 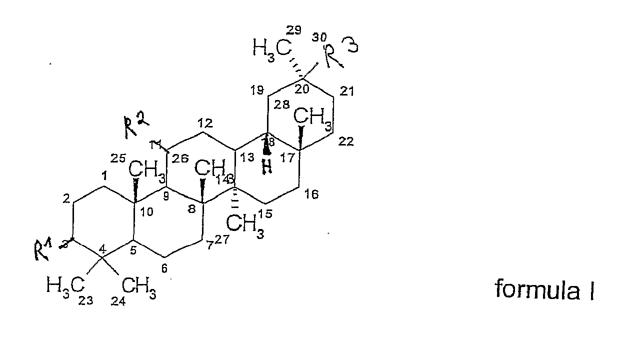 wherein R¹ is
a hydrogen,
a linear or branched C₁-C₁₀ alkyl group,
a linear or branched C₁-C₁₀ alkenyl group,
a linear or branched C₁-C₁₀ alkynyl group,
an ester, amino, halo, hydroxy, carbonyl, carboxy, carboxyphenoxy, C₁-C₄ alkoxy, C₁-C₄ alkoxy carbonyl, C₁-C₄ alkyl amino, di-(C₁-C₄-alkyl) amino, cyano, carboxy amide, carboxy-(C₁-C₄-alkyl)amino, carboxy-di (C₁-C₄-alkyl)sulfo, sulfido (C₁-C₄-alkyl), sulfoxido (C₁-C₄-alkyl), sulfono (C₁-C₄-aminoalkyl) or thio group, a saturated or unsaturated, aromatic or heteroaromatic mono- or polycyclic group,
wherein said cyclic group may be mono- or polysubstituted with an ester, amino, halo, hydroxy, C₁-C₄ alkoxy, carboxy, carbonyl, C₁-C₄ alkoxycarbonyl, carboxyphenoxy, C₁-C₄ alkyl amino, di-(C₁-C₄-alkyl) amino, cyano, carboxy amide, carboxy-(C₁-C₄-alkyl)amino, carboxy-di (C₁-C₄-alkyl)amino, sulfo, sulfido (C₁-C₄-alkyl), sulfoxido (C₁-C₄-alkyl), sulfono (C₁-C₄-alkyl), thio, C₁-C₄ alkyl, C₂-C₄ alkenyl or C₂-C₄ alkynyl group;
R² is a hydrogen, C₁-C₄ alkyl, carbonyl, ester, amino, halo, carbonyl, hydroxy, carboxy, carboxyphenoxy, C₁-C₄ alkoxy, C₁-C₄ alkoxy carbonyl, C₁-C₄ alkyl amino, di-(C₁-C₄-alkyl)amino, cyano, carboxy amide, carboxy-(C₁-C₄-alkyl)amino, carboxy-di(C₁-C₄₋alkyl), sulfo, sulfido (C₁-C₄-alkyl), sulfoxido (C₁-C₄-alkyl), sulfono (C₁-C₄-alkyl) or thio group;
R³ is
a hydrogen,
a linear or branched C₁-C₁₀ alkyl group,
a linear or branched C₁-C₁₀ alkenyl group,
a linear or branched C₁-C₁₀ alkynyl group,
an ester, amino, halo, hydroxy, carbonyl, carboxy, carboxyphenoxy, C₁-C₄ alkoxy, C₁-C₄ alkoxy carbonyl, C₁-C₄ alkyl amino, di-(C₁-C₄-alkyl) amino, cyano, carboxy amide, carboxy-(C₁-C₄-alkyl)amino, carboxy-di
(C₁-C₄-alkyl)sulfo, sulfido (C₁-C₄-alkyl), sulfoxido (C₁-C₄-alkyl), sulfono (C₁-C₄-aminoalkyl) or thio group, a saturated or unsaturated, aromatic or heteroaromatic mono- or polycyclic group;
wherein the chemical bond from carbon 13 to 14 is saturated or unsaturated; or a salt or derivative thereof in the form of an individual enantiomer, diastereomer or a mixture thereof.

The salts of formula I, preferably physiologically accceptable salts, may be obtained in a conventional manner by neutralizing the acids with inorganic or organic bases. Examples of suitable inorganic acids are hydrochloric acid, sulfuric acid, phosphoric acid or hydrobromic acid, and examples of suitable organic acids are carboxylic acid or sulfonic acids such as acetic acid, tartaric acid, lactic acid, propionic acid, glycolic acid, malonic acid, maleic acid, fumaric acid, tannic acid, succinic acid, alginic acid, benzoic acid, 2-phenoxybenzoic acid, 2-acetoxybenzoic acid, cynnamic acid, mandelic acid, citric acid, malic acid, salicylic acid, 3-aminosalicylic acid, ascorbic acid, embonic acid, nicotinic acid, isonicotinic acid, oxalic acid, amino acids, methanesulfonic acid, ethanesulfonic acid, 2-hydroxyethanesulfonic acid, ethane-1,2-disulfonic acid, benzenesulfonic acid, 4-methylbenzenesulfonic acid or naphthalene-2-sulfonic acid. Examples of suitable inorganic bases are sodium hydroxide solution, potassium hydroxide solution, ammonia and suitable organic bases are amines, but preferably tertiary amines such as trimethylamine, triethylamine, pyridine, N,N-dimethylaniline, quinoline, isoquinoline, α-picoline, β-picoline, γ-picoline, quinaldine or pyrimidine.

Physiologically acceptable salts of the compounds of formula I can additionally be obtained by converting derivatives having tertiary amino groups in a manner known per se with quaternizing agents into the corresponding quaternary ammonium salts. Examples of suitable quaternizing agents are alkyl halides such as methyl iodide, ethyl bromide, and N-propyl chloride, but also arylalkyl halides such as benzyl chloride or 2-phenylethyl bromide.

The invention also relates to derivatives of the compounds of formula I which are preferably compounds which are converted, e.g. hydrolized, under physiological conditions to compounds of formula I or into which the compounds of formula I are metabolized under physiological conditions.

The invention further relates to optical enantiomers or diastereomers or mixtures of compounds of formula I which contain an asymmetric carbon atom and in the case of a plurality of asymmetric carbon atoms, also the diastereomeric forms. Compounds of formula I which contain asymmetric carbon atoms and which usually result as racemates can be separated into the optically active isomers in a manner known per se, for example, with an optically active acid. However, it is also possible to employ an optically active starting substance from the outset, in which case a corresponding optically active or diastereomeric compound is obtained as the final product.

In a preferred embodiment of the invention, the 11-β-HSD-type 1 and/or type 2 inhibitors are selected from the group consisting of the formulas 13, 14, 24 and 25 as follows:

| | |
|---|---|
| Formula 13 | 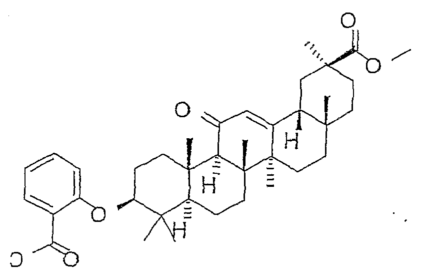 |
| Formula 14 | 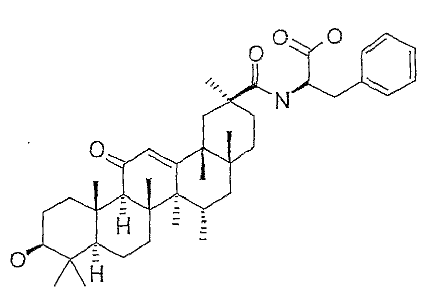 |
| Formula 24 | 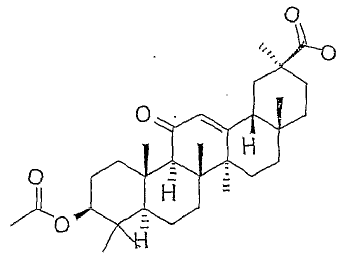 |
| Formula 25 | 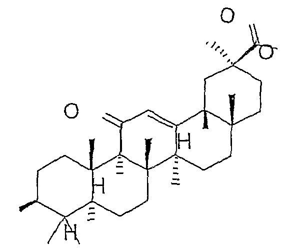 |

Said structures were found to be particularly effective in the specific inhibition of 11-β-HSD, preferably of 11-β-HSD-1, 11-β-HSD-2 and/or 11-β-HSD-1 and 2.

In another preferred embodiment according to the invention, the 11-β-HSD-type and/or type 2 inhibitor has the structure of formula II: 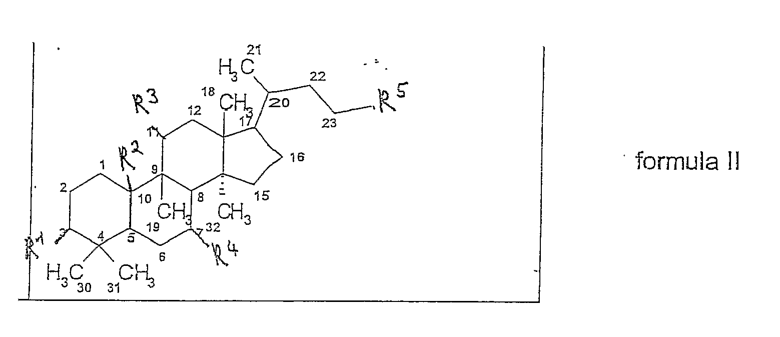 wherein R¹ is
a hydrogen,
a linear or branched C₁-C₁₀ alkyl group,
a linear or branched C₁-C₁₀ alkenyl group,
a linear or branched C₁-C₁₀ alkynyl group,
an ester, amino, halo, hydroxy, carbonyl, carboxy, carboxyphenoxy, C₁-C₄ alkoxy, C₁-C₄ alkoxy carbonyl, C₁-C₄ alkyl amino, di-(C₁-C₄-alkyl) amino, cyano, carboxy amide, carboxy-(C₁-C₄-alkyl)amino, carboxy-di (C₁-C₄-alkyl)sulfo, sulfido (C₁-C₄-alkyl), sulfoxido (C₁-C₄-alkyl), sulfono (C₁-C₄-aminoalkyl), thio group, a saturated or unsaturated, aromatic or heteroaromatic mono- or polycyclic group,
wherein said cyclic group may be mono- or polysubstituted with an ester, amino, halo, hydroxy, C₁-C₄ alkoxy, carbonyl, carboxy, C₁-C₄ alkoxycarbonyl, carboxyphenoxy, C₁-C₄ alkyl amino, di-(C₁-C₄-alkyl) amino, cyano, carboxy amide, carboxy-(C₁-C₄-alkyl)amino, carboxy-di (C₁-C₄-alkyl)amino, sulfo, sulfido (C₁-C₄-alkyl), sulfoxido (C₁-C₄-alkyl), sulfono (C₁-C₄-alkyl), thio, C₁-C₄ alkyl, C₂-C₄ alkenyl or C₂-C₄ alkynyl group;
R² is a hydrogen or C₁-C₄ alkyl,
R³ and R⁴ are each selected from
a hydrogen
a linear or branched C₁-C₁₀ alkyl group,
a linear or branched C₁-C₁₀ alkenyl group,
a linear or branched C₁-C₁₀ alkynyl group,
an ester, amino, halo, hydroxy, carbonyl, carboxy, carboxyphenoxy, C₁-C₄ alkoxy, C₁-C₄ alkoxy carbonyl, C₁-C₄ alkyl amino, di-(C₁-C₄-alkyl) amino, cyano, carboxy amide, carboxy-(C₁-C₄-alkyl)amino, carboxy-di
(C₁-C₄-alkyl)sulfo, sulfido (C₁-C₄-alkyl), sulfoxido (C₁-C₄-alkyl), sulfono (C₁-C₄-aminoalkyl), thio group, a saturated or unsaturated, aromatic or heteroaromatic mono- or polycyclic group;
R⁵ is a hydrogen, C₁-C₄ alky, carbonyl, ester, amino, halo, hydroxy, carboxy, carboxyphenoxy, C₁-C₄ alkoxy, C₁-C₄ alkoxy carbonyl, C₁-C₄ alkyl amino, di-(C₁-C₄-alkyl)amino, cyano, carboxy amide, carboxy-(C₁-C₄-alkyl)amino, carboxy-di(C₁-C₄-alkyl), sulfo, sulfido (C₁-C₄-alkyl), sulfoxido (C₁-C₄-alkyl), sulfono (C₁-C₄-alkyl) or thio group,
wherein the chemical bond from carbon 8 to 9 is saturated or unsaturated;
wherein the chemical bond from carbon 13 to 14 is saturated or unsaturated; or a salt or derivative thereof in the form of an individual enantiomer, diastereomer or a mixture thereof.

The invention of formula II also relates to the above-mentioned physiologically acceptable salts and derivatives of the compound of formula I. Preferably, the structure of formula II is formula 16:

| | |
|---|---|
| Formula 16 | 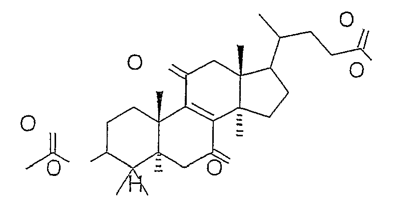 |

In a further preferred embodiment of the invention, the 11-β-HSD-type 1 and/or type 2 inhibitor is formula 7:

| | |
|---|---|
| Formula 7 | 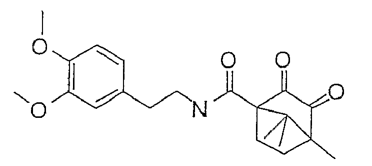 |

Further suitable 11-β-HSD-1 or -2 inhibitors according to the invention used in the prevention and/or treatment of inflammation-induced and/or immune-mediated bone loss, for example, are, but not limited to, 18-β-glycyrrhetinic acid, progesterone, 5α-dihydroprogesterone, 5β-dihydroprogesterone, 20α-dihydroprogesterone, 3β5α-tetrahydroprogesterone, 17α-OH-progesterone, 20a-dihydro-5α-dihydroprogesterone, 20α-Dihydroprogesterone, 11α-OH-progesterone, 11β-OH-progesterone, corticosterone, 11β-OH-androstenoidone, 3-alpha, 5-beta-tetrahydroprogesterone, 3-alpha, 5-beta-tetrahydro-11-deoxy-corticosterone, 11-epicortisol, chenodeoxycholic acid, cholic acid, glycyrrhetinic acid (3β-hydroxy-11-oxooteane-12-ene-30-acid) and derivatives thereof such as glycyrrhicine, glycyrrhicinic acid and carbenoxolone; furosemide and derivatives thereof, flavonoides and derivatives thereof such as naringenine, triterpinoides (e.g. CHAPS), ketokonazole, saiboku-to, gossypol, metyrapone, 11-epiprednisolone. Further suitable inhibitors are steroid-like, such as dexamethasone, budesonide, deflazacort and stanozolol. Further suitable inhibitors are those described in patent applications WO 02/072084 A2, WO 03/043999 A1 as well as WO 03/044000 A1.Thus, suitable inhibitors, particularly, are compounds of formula **formula III or a salt thereof:** 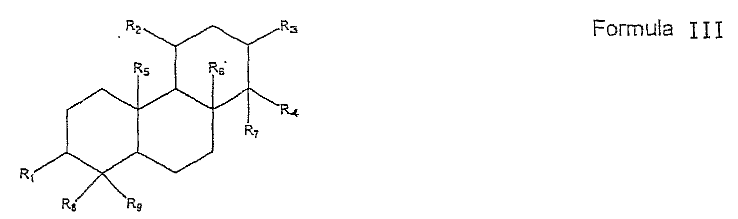 wherein R1 is selected from H, alkyl, cycloalkyl, alkenyl, aryl, =O, OH, O-alkyl, O-acyl and O-aryl; and R2 is selected from H, =O, OH, hydrocarbyl, oxyhydrocarbyl, and halo;
R5 to R9 are independently selected from H and hydrocarbyl;
R3 and R4 together represent
(i) a group of formula II 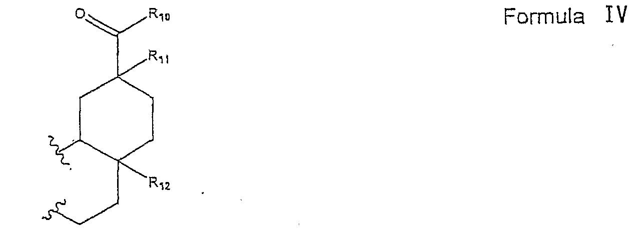 wherein R10 is selected from OH, hydrocarbyl, N-hydrocarbyl and O-hydrocarbyl;
   wherein when R1 is OH, R10 is hydrocarbyl, N-hydrocarbyl or O-hydrocarbyl; R11 and R12 are independently selected from H and hydrocarbyl, or
(ii) a group of formula V 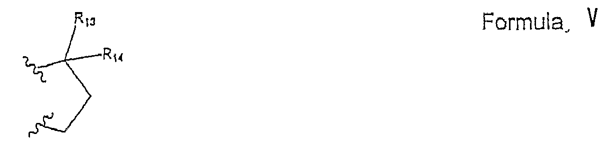 wherein R13 is hydrocarbyl and R14 is H or OH, or R13 and R14 together represent =O.

Further suitable inhibitors are compounds of formula VI 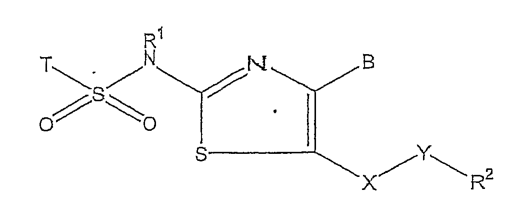 wherein
T is an aryl ring or heteroaryl ring, optionally independently substituted by [R]ₙ, wherein n is an integer 0-5, and R is hydrogen, aryl, heteroaryl, a heterocyclic ring, optionally halogenated C₁₋₆-alkyl, optionally halogenated C₁₋₆-alkoxy, C₁₋₆-alkylsulfonyl, carboxy, cyano, nitro, halogen, aryloxy, arylsulfonyl, arylamino, wherein aryl, heteroaryl and aryloxy residues and heterocyclic rings are further optionally substituted in one or more positions independently of each other by C₁₋₆-acyl, C₁₋₆-alkylthio, cyano, nitro, hydrogen, halogen, optionally halogenated C₁₋₆-alkyl, optionally halogenated C₁₋₆-alkoxy, amide which is optionally mono- or di-substituted, (benzoylamino)methyl, carboxy, 2-thienylmethylamino or ({[4-(2-ethoxy-2-oxoethyl)-1,3 -thiazol-2-yl]amino}carbonyl); or T is selected from 5-(dimethylamino)-1-naphthyl and phenyl substituted with one or more of benzeneamino, benzylamino, 3-pyridylmethylamino and 2-thienylmethylamino;
R¹ is hydrogen or C₁₋₆-alkyl;
X is CH₂ or CO;
Y is CH₂, CO or a single bond;
B is hydrogen, C₁₋₆-alkyl or dimethylaminomethyl;
R² is selected from C₁₋₆-alkyl, azido, arylthio, heteroarylthio, halogen, hydroxymethyl, 2-hydroxyethylaminomethyl, methylsulfonyloxymethyl, 3-oxo-4-morpholinolinylmethylene, C₁₋₆-alkoxycarbonyl, 5-methyl-1,3,4-oxadiazol-2-yl;
NR³R⁴, wherein R³ and R⁴ are each independently selected from hydrogen, ethyl, isopropyl, n-propyl, optionally halogenated C₁₋₆-alkylsulfonyl, C₁₋₆-alkoxy, 2-methoxyethyl, 2-hydroxyethyl, 1-methylimidazolylsulfonyl, C₁₋₆-acyl, cyclohexylmethyl, cyclopropanecarbonyl, aryl, optionally halogenated arylsulfonyl, furylcarbonyl, tetrahydro-2-furanybnethyl, N-carbethoxypiperidyl, or C₁₋₆alkyl substituted with one or more aryl, heterocyclic or heteroaryl, or
NR³R⁴ represent together heterocyclic systems which are imidazole, piperidine, pyrrolidine, piperazine, morpholine, oxazepine, oxazole, thiomorpholine, 1,1-dioxidothiomorpholine, 2-(3,4-dihydro-2(1H)isoquinolinyl), or (1S,4S)-2-oxa-5-azabicyclo[2.2.1]hept-5-yl, which heterocyclic systems are optionally substituted by C₁₋₆-alkyl, C₁₋₆-acyl, hydroxy, oxo, t-butoxycarbonyl;
OCONR³R⁴, wherein R³ and R⁴ are each independently selected from hydrogen, C₁₋₆-alkyl or form together with the N-atom to which they are attached morpholinyl; R⁵O, wherein R⁵ is hydrogen, optionally halogenated C₁₋₆-alkyl, aryl, heteroaryl, C₁₋₆-acyl, C₁₋₆-alkylsulfonyl, arylcarbonyl, heteroarylcarbonyl, 2-carbomethoxyphenyl;
or a salt, hydrate or solvate thereof; with the proviso that when: X is CH₂, Y is CH₂, then R² is not methyl, ethyl, diethylamino, 1-pyrrolidinyl, and 1-piperidinyl;
X is CH₂, Y is CH₂, R² is morpholinyl, then T is not 4-methylphenyl;
X is CH₂, Y is CO, then R² is not hydroxy;
X is CH₂, Y is a single bond, then R² is not ethyl, n-propyl;
X is CH₂, Y is a single bond, R² is methyl, B is methyl, then T is not 3-chloro-2-methylphenyl;
X is CO, Y is a single bond, then R² is not methyl;
X is CO, Y is a single bond, R² is ethoxy, B is methyl, then T is not 3-chloro-2-methylphenyl, 1,1'-biphenyl-4-yl, 4-n-propylphenyl, 2,4-dichloro-6-methylphenyl, and 2,4,6-trichlorophenyl.

Also suited compounds are compounds of formula VII: 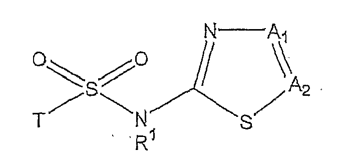 wherein:
T is an aryl ring or heteroaryl ring, optionally independently substituted by [R]ₙ,
wherein n is an integer 0-5, and R is hydrogen, aryl, heteroaryl, a heterocyclic ring, optionally halogenated C₁₋₆-alkyl, optionally halogenated C₁₋₆-alkoxy, C₁₋₆-alkylsulfonyl, carboxy, cyano, nitro, halogen, amine which is mono- or di-substituted, amide which is optionally mono- or di-substituted, aryloxy, arylsulfonyl, arylamino, wherein aryl, heteroaryl and aryloxy residues and heterocyclic rings are further optionally substituted in one or more positions independently of each other by C₁₋₆-acyl, C₁₋₆-alkylthio, cyano, nitro, hydrogen, halogen, optionally halogenated C₁₋₆-alkyl, optionally halogenated C₁₋₆-alkoxy, amide which is optionally mono- or di-substituted, (benzoylamino)methyl, carboxy, 2-thienylmethylamino or ({[4-(2-ethoxy-2-oxoethyl)-1,3-thiazol-2-yl]amino}carbonyl);
R¹ is hydrogen or C₁₋₆-akyl;
A₁ and A₂ are a nitrogen atom or C-Z, provided that A₁ and A₂ have different meanings, wherein:
- Z is selected from an aryl ring or heteroaryl ring, which is further optionally substituted in one or more positions independently of each other by hydrogen, C₁₋₆-alkyl, halogenated C₁₋₆-alkyl, halogen, C₁₋₆-alkoxy, nitro, C₁₋₆-alkoxycarbonyl, C₁₋₆-alkylsulfonyl, acetylamino or aryloxy, wherein the aryloxy is further optionally substituted in one or more positions independently of each other by hydrogen and halogen; or is X-Y-R², wherein
- X is CH₂ or CO;
- Y is CH₂, CO or a single bond;
- R² is selected from C₁₋₆-alkyl, azido, arylthio, heteroarylthio, halogen, hydroxymethyl, 2-hydroxyethylaminomethyl, methylsulfonyloxymethyl, 3-oxo-4-morpholinolinylmethylene, C₁₋₆-alkoxycarbonyl, 5-methyl-1,3,4-oxadiazol-2-yl; NR³R⁴, wherein R³ and R⁴ are each independently selected from hydrogen, C₁₋₆-alkyl, optionally halogenated C₁₋₆-alkylsulfonyl, C₁₋₆-alkoxy, 2-methoxyethyl, 2-hydroxyethyl, 1-methylimidazolylsulfonyl, C₁₋₆-acyl, cyclohexylmethyl, cyclopropanecarbonyl, aryl, optionally halogenated arylsulfonyl, furylcarbonyl, tetrahydro-2-furanylmethyl, N-carbethoxypiperidyl, or C₁₋₆-alkyl substituted with one or more aryl, heterocyclic or heteroaryl, or
   NR³R⁴ represent together heterocyclic systems which are imidazole, piperidine, pyrrolidine, piperazine, morpholine, oxazepine, oxazole, thiomorpholine, 1,1-dioxidothiomorpholine, 2-(3,4-dihydro-2(1H)isoquinolinyl), or (1S,4S)-2-oxa-5-azabicyclo[2.2.1]hept-5-yl, which heterocyclic systems are optionally substituted by C₁₋₆-alkyl, C₁₋₆-acyl, hydroxy, oxo, t-butoxycarbonyl; OCONR³R⁴, wherein R³ and R⁴ are each independently selected from hydrogen, C₁₋₆-alkyl or form together with the N-atom to which they are attached morpholinyl; R⁵O, wherein R⁵ is hydrogen, optionally halogenated C₁₋₆-alkyl, aryl, heteroaryl, C₁₋₆-acyl, C₁₋₆-alkylsulfonyl, arylcarbonyl, heteroarylcarbonyl, 2-carbomethoxyphenyl;
   or a salt, hydrate or solvate thereof;
   with the proviso that when:
   A₁ is C-Z and A₂ is a nitrogen atom, then T is not phenyl only substituted with a
   nitrogen containing substituent in position 4 with a nitrogen atom closest to the phenyl ring, is not phenyl only substituted with methyl in position 2, is not phenyl only substituted with methyl in position 4, and is not phenyl only substituted with ethyl in position 4;
   A₁ is a nitrogen atom and A₂ is C-Z, then Z is not 2-furyl, 5-nitro-2-furyl, 2-thienyl, optionally substituted phenyl, para-substituted benzyl;
   A₁ is a nitrogen atom and A₂ is C-Z, X is CH₂, Y is a single bond, then R² is not C₁₋₆-alkyl, methoxy, ethoxy, benzothiazol-2-ylthio and NR³R⁴, wherein R³ and R⁴ are selected from methyl, ethyl, n-propyl, n-butyl;
   A₁ is a nitrogen atom and A₂ is C-Z, X is CH₂, Y is CH₂, then R² is not C₁₋₆-alkyl and NR³R⁴, wherein R³ and R⁴ are selected from methyl, ethyl, n-propyl, n-butyl.

Further preferred structures are those shown in Fig. 1

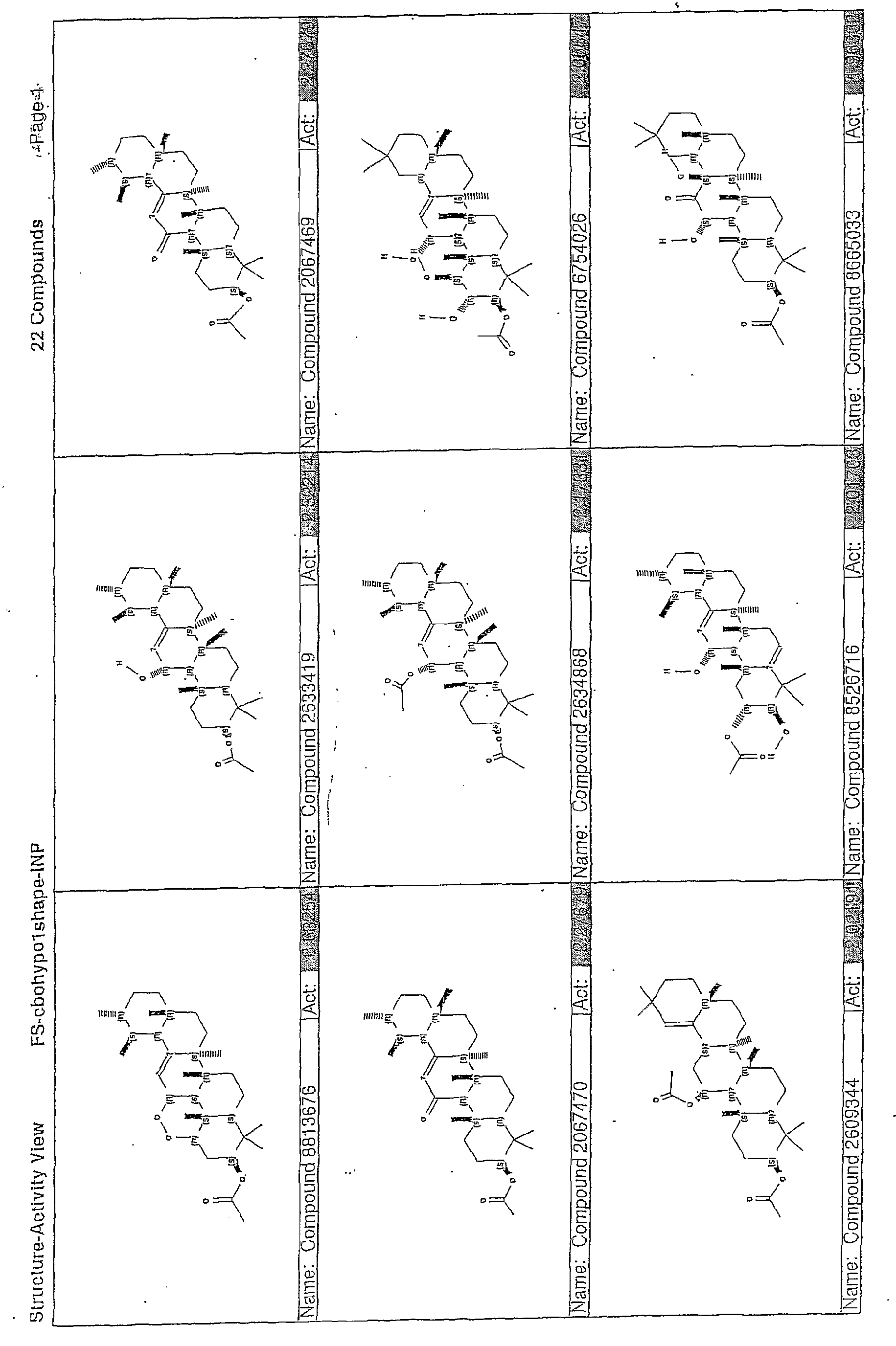

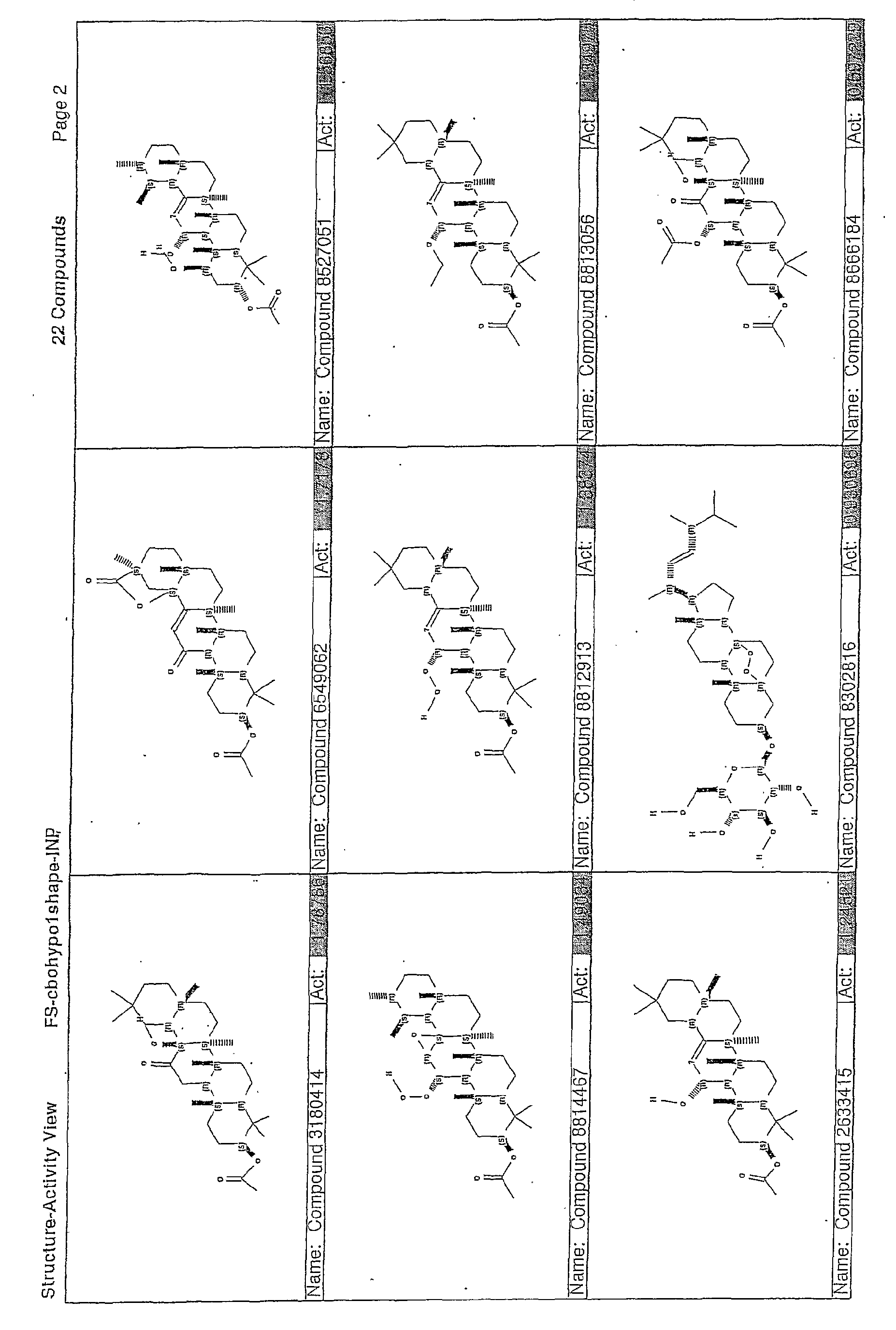

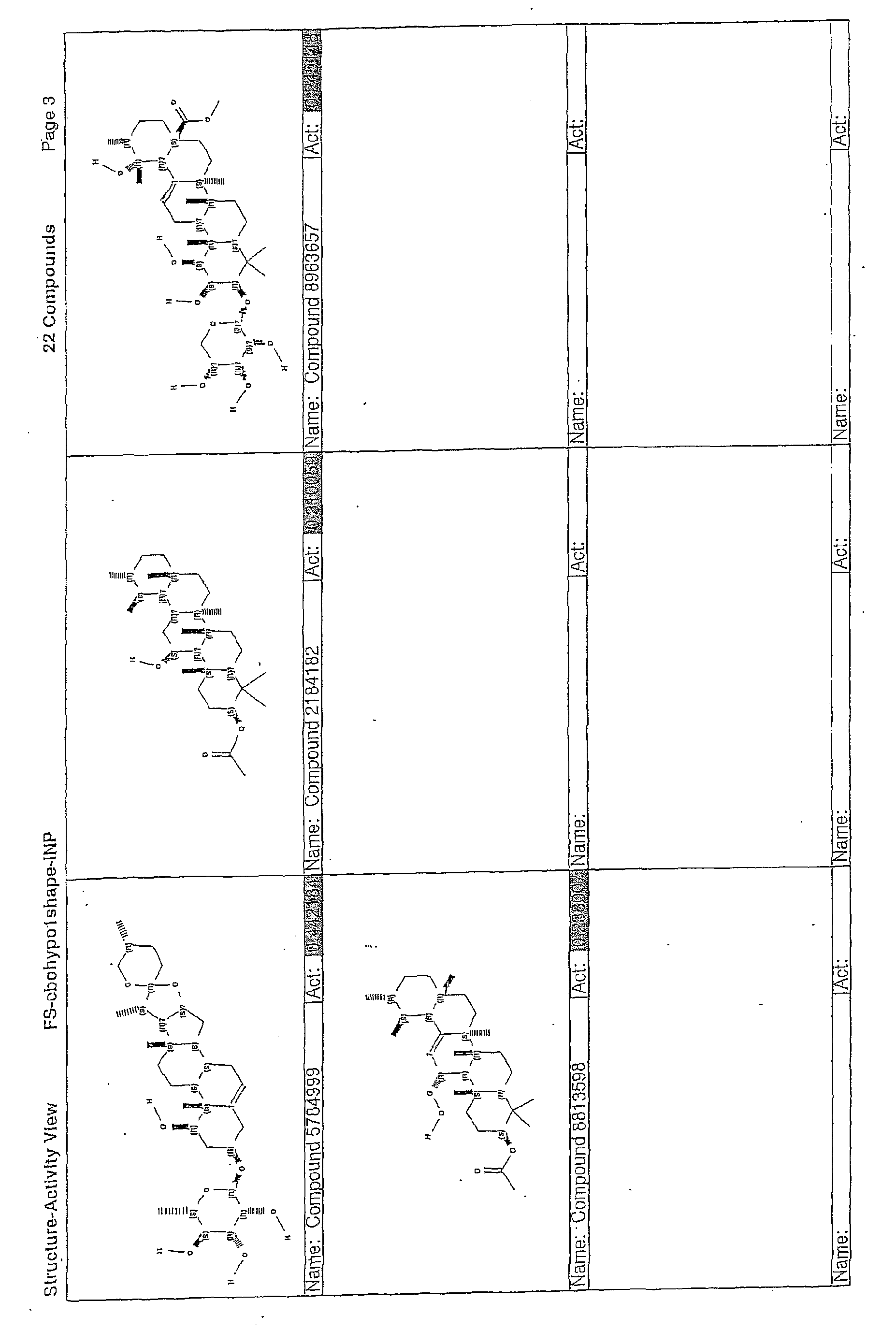

Particularly preferably, the inhibitors are selected from 3-chloro-2-methyl-N-{4-[2-(4-methyl-1-piperazinyl)-2-oxoethyl]-1,3-thiazol-2-yl}benzenesulfonamide and 2-(2-{[(3-chloro-2-methylphenyl)sulfonyl]amino}-1,3-thiazol-4-yl)-N,N-diethylacetamide.

Further suitable inhibitors are those bicyclo[2.2.2]-oct-1-yl-1,2,4-triazole derivatives described in Patent Application WO 2004/058741. Hence, suitable inhibitors, in particular, are compounds of formula VIII: 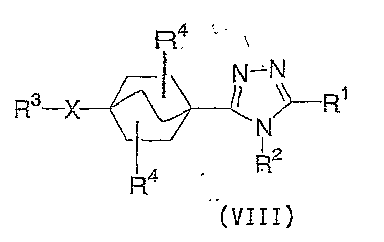 or a pharmaceutically acceptable salt thereof; wherein
each p is independently 0, 1, or 2;
each n is independently 0, 1, or 2;
X is selected from the group consisting of a single bond, O, S(O)p, NR⁶, 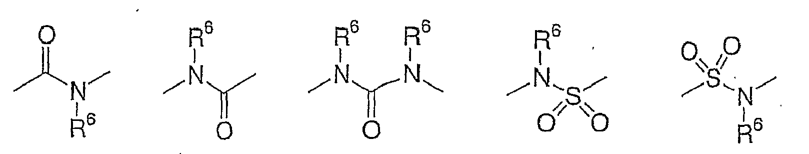 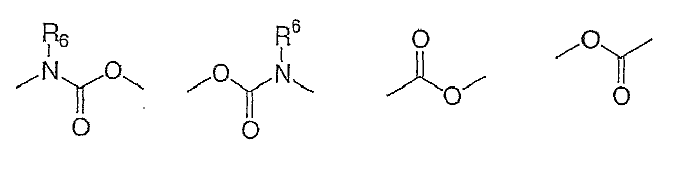 R¹ is selected from the group consisting of
arylcarbonyl,
(CH₂)ₙ-aryl, and
(CH₂)ₙ-heteroaryl;
in which aryl and heteroaryl are unsubstituted or substituted with one to three substituents independently selected from R⁵;
R2 is selected from the group consisting of
hydrogen,
C₁₋₈ alkyl,
C₂₋₆ alkenyl, and
(CH₂)ₙ-C₃₋₆ cycloalkyl,
in which alkyl, alkenyl, and cycloalkyl are unsubstituted or substituted with one to three substituents independently selected from R⁸ and oxo;
each R⁴ is independently selected from the group consisting of
hydrogen,
halogen,
hydroxy,
oxo,
C₁₋₃ alkyl, and
C₁₋₃ alkoxy;
R3 is selected from the group consisting of
hydrogen,
C₁₋₁₀ alkyl,
C₂₋₁₀ alkenyl,
(CH2)ₙ-C₃₋₆ cycloalkyl,
(CH₂)ₙ-aryl,
(CH₂)ₙ-heteroaryl, and
(CH₂)ₙ-heterocyclyl;
in which aryl, heteroaryl, and heterocyclyl are unsubstituted or substituted with one to three
substituents independently selected from R⁵; and alkyl, alkenyl, and cycloalkyl are unsubstituted or substituted with one to five groups independently selected from R⁸ and oxo;
R⁵ and R⁸ are each independently selected from the group consisting of
hydrogen,
formyl,
C₁₋₆ alkyl,
(CH₂)ₙ-aryl,
(CH₂)ₙ-heteroaryl,
(CH₂)ₙ-heterocyclyl,
(CH₂)ₙC₃₋₇ cycloalkyl,
halogen,
OR⁷,
(CH₂)ₙN(R⁷)₂,
cyano,
(CH₂)ₙCO₂R⁷,
NO₂,
(CH₂)ₙNR⁷SO₂R⁶,
(CH₂)ₙSO₂N(R⁷)₂,
(CH₂)ₙS(O)ₚR⁶,
(CH₂)ₙSO₂OR⁷,
(CH₂)ₙNR⁷C(O)NCR⁷)₂,
(CH₂)ₙC(O)N(R⁷)₂,
(CH₂)ₙNR₆C(O)R⁶,
(CH₂)ₙNR₆CO₂R⁷,
O(CH₂)ₙC(O)N(R⁷)₂,
CF3,
CH₂CF₃,
OCF3,
OCHCF₂, and
OCH₂CF₃;
wherein aryl, heteroaryl, cycloalkyl, and heterocyclyl are unsubstituted or substituted with one to three substituents independently selected from halogen, hydroxy, C₁₋₄ alkyl, trifluoromethyl, trifluoromethoxy, and C₁₋₄ alkoxy; and wherein any methylene (CH₂) carbon atom in R⁵ and
R⁸ is unsubstituted or substituted with one to two groups independently selected from halogen, hydroxy, and C₁₋₄ alkyl; or two substituents when on the same methylene (CH₂) carbon atom are taken together with the carbon atom to which they are attached to form a cyclopropyl group; each R⁶ is independently selected from the group consisting of
C₁₋₈ alkyl,
(CH₂)ₙ-aryl,
(CH₂)ₙ-heteroaryl, and
(CH₂)ₙC₃₋₇ cycloalkyl;
wherein alkyl and cycloalkyl are unsubstituted or substituted with one to five substituents independently selected from halogen, oxo, C₁₋₄ alkoxy, C₁₋₄ alkylthio, hydroxy, amino; and aryl and heteroaryl are unsubstituted or substituted with one to three substituents independently selected from cyano, halogen, hydroxy, amino, carboxy, trifluoromethyl, trifluoromethoxy, C₁₋₄ alkyl, and C₁₋₄ alkoxy; or two R⁶ groups together with the atom to which they are attached form a 5- to 8-membered mono- or bicyclic ring system optionally containing an additional heteroatom selected from O, S, and NC₁₋₄ alkyl; and each R⁷ is hydrogen or R⁶.

Further suitable inhibitors are those disclosed in Patent Application U.S. 6,730,690, U.S. 2004/0106664 as well as WO 03/104208. Thus, suitable inhibitors, particularly, are compounds of formula IX: 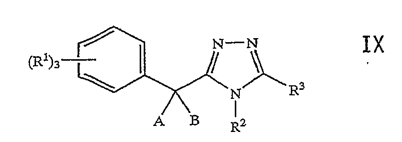 or a pharmaceutically acceptable salt or solvate thereof,
wherein:
A and B may be taken separately or together; when taken separately,
A represents halo, C₁₋₆alkyl, OC₁₋₆alkyl or phenyl, said alkyl, phenyl and the alkyl portion of OC₁₋₆alkyl being optionally substituted with 1-3 halo groups; and
B represents represents H, halo, C₁₋₆alkyl, -OC₁₋₆alkyl, -SC₁₋₆alkyl, C₂₋₆alkenyl, phenyl or naphthyl, said alkyl, alkenyl, phenyl, naphthyl, and the alkyl portions of -OC₁₋₆alkyl and -SC₁₋₆alkyl being optionally substituted with 1-3 groups selected from halo, OH, CH₃O, CF₃ and OCF₃; and when taken together,
A and B together represents (a) C₁₋₄alkylene optionally substituted with 1-3 halo groups, and 1-2 R*^{a}* groups wherein R*^{a}* represents C₁₋₃alkyl, OC₁₋₃alkyl, C₆₋₁₀arC₁₋₆alkylene or phenyl optionally substituted with 1-3 halo groups, or (b) C₂₋₅alkanediyl such that a 3-6 membered ring is formed with the carbon atom to which they are attached, said ring being optionally interrupted with 1 double bond or 1-2 heteroatoms selected from O, S and N, said 3-6 membered ring being optionally substituted with C₁₋₄alkylene oxo, ethylenedioxy or propylenedioxy, and being further optionally substituted with 1-4 groups selected from halo, C₁₋₄alkyl, haloC₁₋₄alkyl, C₁₋₃acyl, C₁₋₃acyloxy, C₁₋₃alkoxy, C₁₋₆alkylOC(O)-, C₂₋₄alkenyl, C₂₋₄alkynyl, C₁₋₃alkoxyC₁₋₃alkyl, C₁₋₃alkoxyC₁₋₃alkoxy, phenyl, CN, OH, D, NH₂, NHR*^{a}* and N(R*^{a}*)₂
   wherein R*^{a}* is as previously defined; each R¹ represents H or is independently selected from the group consisting of: OH, halo, C₁₋₁₀alkyl, C₁₋₆alkoxy and C₆₋₁₀aryl, said C₁₋₁₀alkyl, C₆₋₁₀aryl and the alkyl portion of C₁₋₆alkoxy being optionally substituted with 1-3 halo, OH, OC₁₋₃alkyl, phenyl or naphthyl groups, said phenyl and naphthyl being optionally substituted with 1-3 substituents independently selected from halo, OCH₃, OCF₃, CH₃, CF₃ and phenyl, wherein said phenyl is optionally substituted with 1-3 halo groups, or two R³ groups taken together represent a fused
   C₅₋₆alkyl or aryl ring, which may be optionally substituted with 1-2 OH or R*^{a}* groups, wherein R*^{a}* is as defined above;
   R² and R³ are taken together or separately;
   when taken together, R² and R³ represent (a) a C₃₋₈ alkanediyl forming a fused 5-10 membered non-aromatic ring optionally interrupted with 1-2 double bonds, and optionally interrupted by 1-2 heteroatoms selected from O, S and N; or (b) a fused 6-10 membered aromatic monocyclic or bicyclic group, said alkanediyl and aromatic monocyclic or bicyclic group being optionally substituted with 1-6 halo atoms, and 1-4 of OH, C₁₋₃alkyl, OC₁₋₃alkyl, haloC₁₋₃alkyl, haloC₁₋₃alkoxy, and phenyl, said phenyl being optionally substituted with 1-4 groups independently selected from halo, C₁₋₃alkyl, OC₁₋₃alkyl, and said C₁₋₃alkyl and the
   C₁₋₃alkyl portion of OC₁₋₃alkyl being optionally substituted with 1-3 halo groups;
   when taken separately,
   R² is selected from the group consisting of: (a) C₁₋₁₄alkyl optionally substituted with 1-6 halo groups and 1-3 substituents selected from OH, OC₁₋₃alkyl, and phenyl, said phenyl being optionally substituted with 1-4 groups independently selected from halo, OCH₃, OCF₃, CH₃ and CF₃, and said C₁₋₃alkyl portion of OC₁₋₃alkyl being optionally substituted with 1-3 halo groups; (b) phenyl or pyridyl optionally substituted with 1-3 halo, OH or R*^{a}* groups, with R*^{a}* as previously defined; (c) C₂₋₁₀ alkenyl, optionally substituted with 1-3 substituents independently selected from halo, OH and OC₁₋₃alkyl, said C₁₋₃alkyl portion of OC₁₋₃alkyl being optionally substituted with 1-3 halo groups; (d)
   CH₂CO₂H; (e) CH₂CO₂C₁₋₆alkyl; (f) CR₂C(O)NHR*^{a}*
   wherein R*^{a}* is as previously defined; (g) NH₂, NHR*^{a}* and N(R*^{a}*)₂ wherein R*^{a}* is as previously defined; and R³ is selected from the group consisting of: C₁₋₁₄alkyl, C₂₋₁₀alkenyl, SC₁₋₆alkyl, C₆₋₁₀aryl, heterocyclyl and heteroaryl, said alkyl, alkenyl, aryl, heterocyclyl, heteroaryl and the alkyl portion of SC₁₋₆alkyl being optionally substituted with (a) R; (b) 1-6 halo groups and (c) 1-3 groups selected from OH, NH₂, NHC₁₋₄alkyl, N(C₁₋₄alkyl)₂, C₁₋₄alkyl, OC₁₋₄alkyl, CN, C₁₋₄alk-ylS(O)*ₓ*- wherein x is 0, 1 or 2, C₁₋₄alkylSO₂NH-, H₂NSO₂-, C₁₋₄alkylNHSO₂-and (C₁₋₄alkyl)₂NSO₂-, said C₁₋₄alkyl and the C₁₋₄alkyl portions of said groups being optionally substituted with phenyl and 1-3 halo groups, and
   R is selected from heterocyclyl, heteroaryl and aryl, said group being optionally substituted with 1-4 groups selected from halo, C₁₋₄alkyl, C₁₋₄alkylS(O)*ₓ*-, with x as previously defined, C₁₋₄ alkylSO₂NH-, H₂NSO₂-, C₁₋₄alkylNHNHSO₂-, (C₁₋₄ alkyl)₂NSO₂-, CN, OH, OC₁₋₄alkyl, and, said C₁₋₄alkyl and the C₁₋₄alkyl portions of said groups being optionally substituted with 1-5 halo and 1 group selected from OH and OC₁₋₃alkyl.

Further suitable 11-β-HSD inhibitors are those described in Patent Application WO 03/065983. Therefore, suitable inhibitors, in particular, are compounds of formula X or a salt thereof: 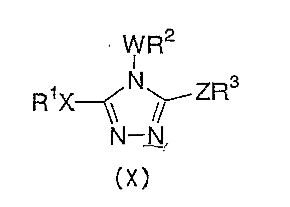 In formula X.
R¹ is adamantyl, unsubstituted or substituted with one to five substituents independently selected from halogen, OCH₃, OCF₃, CH₃, CF₃, and phenyl, wherein said phenyl is unsubstituted or substituted with one to three halogens;
W is selected from the group consisting of NR^{a} and a single bond;
X is selected from the group consisting of CH₂ and a single bond;
Z is selected from the group consisting of S and a single bond;
R^{a} is selected from the group consisting of hydrogen and C₁₋₆ alkyl, wherein alkyl is unsubstituted or substituted with one to five fluorines;
R2 is selected from the group consisting of
hydrogen,
C₁₋₁₀ alkyl, unsubstituted or substituted with one to six substituents independently selected from zero to five halogens and zero or one group selected from hydroxy and C₁₋₃ alkoxy, said alkoxy group being unsubstituted or substituted with one to three halogens,
C₂₋₁₀ alkenyl, unsubstituted or substituted with one to six substituents independently selected from zero to five halogens and zero or one group selected from hydroxy and C₁₋₃ alkoxy, said alkoxy group being unsubstituted or substituted with one to three halogens,
CH₂CO₂H,
CH₂CO₂C₁₋₆ alkyl,
CH₂CONHR^{a},
(CH₂)₀₋₂C₃₋₉ cycloalkyl,
(CH₂)₀₋₂C₅₋₁₂ bicycloalkyl,
(CH₂)₀₋₂adamantyl, and
(CH₂)₀₋₂R;
wherein said C₃₋₉ cycloalkyl and C₅₋₁₂ bicycloalkyl optionally have one to two double bonds, and said C₃₋₉ cycloalkyl, C₅₋₁₂ bicycloalkyl, and adamantyl are unsubstituted or substituted with one to six substituents independently selected from (a) zero to five halogens, CH₃, CF₃, OCH₃, and OCF₃, and (b) zero or one phenyl, said phenyl being unsubstituted or substituted with one to four groups independently selected from halogen, OCH₃, OCF₃, CH₃, and CF₃;
R3 is selected from the group consisting of hydrogen,
C₁₋₁₀ alkyl, unsubstituted or substituted with one to six substituents independently selected from zero to five halogens and zero or one group selected from hydroxy and C₁₋₃ alkoxy, said alkoxy group being unsubstituted or substituted with one to three halogens,
C₂₋₁₀ alkenyl, unsubstituted or substituted with one to six substituents independently selected from zero to five halogens and zero or one group selected from hydroxy and C₁₋₃ alkoxy, said alkoxy group being unsubstituted or substituted with one to three halogens,
YC₃₋₉ cycloalkyl,
YC₅₋₁₂ bicycloalkyl,
Y adamantyl, and
YR;
wherein said C₃₋₉ cycloalkyl and C₅₋₁₂ bicycloalkyl optionally have one to two double bonds, and said C₃₋₉ cycloalkyl, C₅₋₁₂ bicycloalkyl, and adamantyl are unsubstituted or substituted with one to six substituents independently selected from (a) zero to five halogens, CH₃, CF₃, OCH₃, and OCF₃, and (b) zero or one phenyl, said phenyl being unsubstituted or substituted with one to four groups independently selected from halogen, OCH₃, OCF₃, CH³, and CF³;
R is selected from the group consisting of benzodioxolane, furan, tetrahydrofuran, thiophene, tetrahydrothiophene, dihydropyran, tetrahydropyran, pyridine, piperidine, benzofuran, dihydrobenzofuran, benzothiophene, dihydrobenzothiophene, indole, dihydroindole, indene, indane, 1,3-dioxolane, 1,3-dioxane, phenyl, and naphthyl; wherein R is unsubstituted or substituted with one to four groups independently selected from halogen, C₁₋₄ alkylthio, C₁₋₄ alkylsulfinyl, C₁₋₄ alkylsulfonyl, C₂₋₄ alkenylsulfonyl, CN, OH, OCH₃, OCF₃, and C₁₋₄ alkyl, said C₁₋₄ alkyl being unsubstituted or substituted with one to five halogens or one substituent selected from OH and C₁₋₃ alkoxy; and
Y is selected from (CH₂)₀₋₂ and (-HC=CH-);
or alternatively R² and R³ taken together form a bridging group R⁴, providing a compound of structural formula Ia: 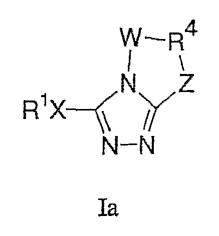 wherein R⁴ is
a C₂₋₈ alkylene group, optionally containing one heteroatom selected from O and NRb between two adjacent carbon atoms of said C₂₋₈ alkylene group, optionally containing one to two carbon-carbon double bonds when R⁴ is a C₃₋₈ alkylene group, and optionally also comprising a carbon-carbon single bond connecting two non-adjacent carbon atoms of said C₂₋₈ alkylene group, or
a C₄₋₈ cycloalkyl group;
wherein R^{b} is selected from the group consisting of hydrogen and C₁₋₆ alkyl, unsubstituted or substituted with one to six substituents independently selected from zero to five fluorines and zero or one phenyl, said phenyl being unsubstituted or substituted with one to three substituents independently selected from halogen, CH₃, CF₃, OCH₃, and OCF₃;
wherein R⁴ is unsubstituted or substituted with one to five R^{C} substituents, wherein each R^{C} is independently selected from halogen, OH, OCH₃, OCF₃, C₁₋₆ alkyl, C₂₋₆ alkenyl, phenyl, biphenyl, C₃₋₈ cycloalkyl, C₁₋₆ alkyloxycarbonyl, an epoxide group bridging 2 adjacent carbons, and 1,3-dioxolanyl geminally disubstituted onto one carbon of R⁴, wherein each C₁₋₆ alkyl and C₂₋₆ alkenyl is unsubstituted or substituted with one to five substituents independently selected from zero to three halogens and zero to two groups selected from phenyl, C₁₋₆ alkyloxycarbonyl, 1,3-dioxolanyl geminally disubstituted onto one carbon, and CN, and wherein each phenyl, biphenyl, and C₃₋₈ cycloalkyl, either as R^{c} or as a substituent on R^{c}, is unsubstituted or substituted with one to three groups independently selected from halogen, CH₃, CF₃, OCH₃, and OCF₃;
wherein R⁴ optionally has a fused phenyl ring, a benzodioxinyl ring, or a dihydrobenzodioxinyl ring, said phenyl ring, benzodioxinyl ring, and dihydrobenzodioxinyl ring being unsubstituted or substituted with one to three substituents independently selected from halogen, CH₃, CF₃, OCH₃, and OCF₃; and
wherein R⁴, including said optional fused phenyl ring, benzodioxinyl ring, or dihydrobenzodioxinyl ring and including all substituents on R⁴ and said fused phenyl ring, benzodioxinyl ring, or dihydrobenzodioxinyl ring, has no more than 20 carbon atoms; Other suitable inhibitors are those described in Patent Application WO 2004/027042. Hence, suitable inhibitors, particularly, are compounds of formulas XI, XII, XIII, XIV, XV, XVI, XVII and XVIII or a salt thereof: 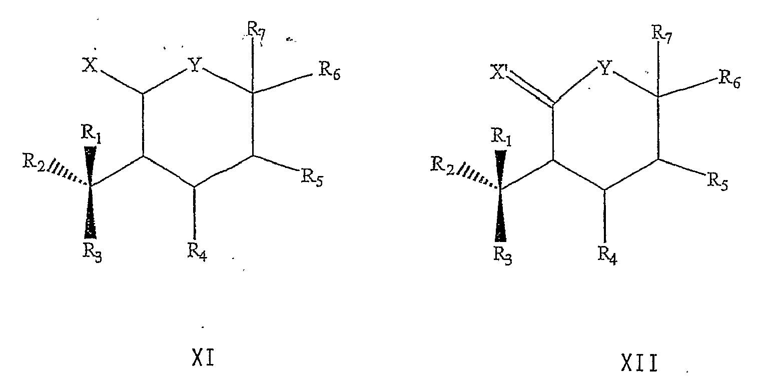 wherein R₁ is H or CH₃, R₂ is H, CH₃, or CH₂CH₃, R₃ is H, CH₃, CH₂CH₃, or CH₂CH₂CH₃, R₄ is H, CH₃, or CH₂CH₃, R₅ is H, CH₃, or CH₂CH₃, R₆ is H, CH₃, CH₂CH₃, or CH₂CH₂CH₃, R₇ is H or CH₃, X is OH, SH, or NH₂, X' is O, S, or NH, and Y is O, S, NH, or CH₂. 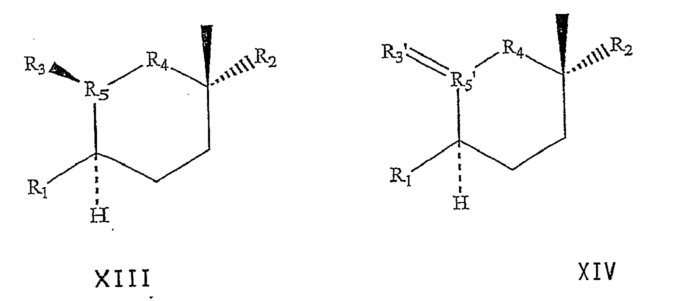 wherein R₁ is 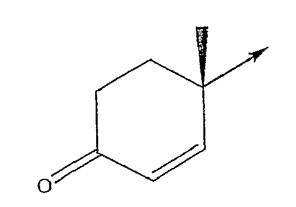 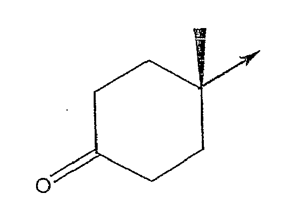 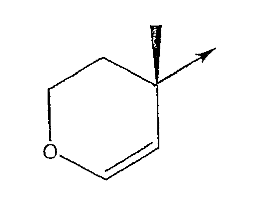 or 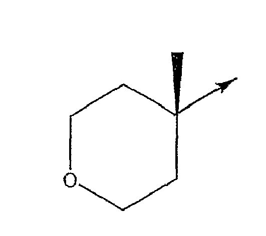 R₂ is 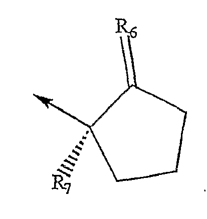 wherein R₆ is O or S and R₇ is H, OH, or halogen, or 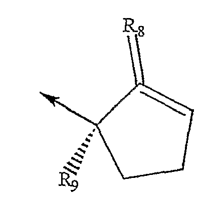 wherein R₈ is H, OH, or halogen, and R₉ is H, OH, or halogen, and R₃ is OH, SH, or NH₂, R₃' is O, S, or NH, R₄ is O, S, NH, or CH₂, R₅ is N or CH₂, and R₅' is SO or CH₂. 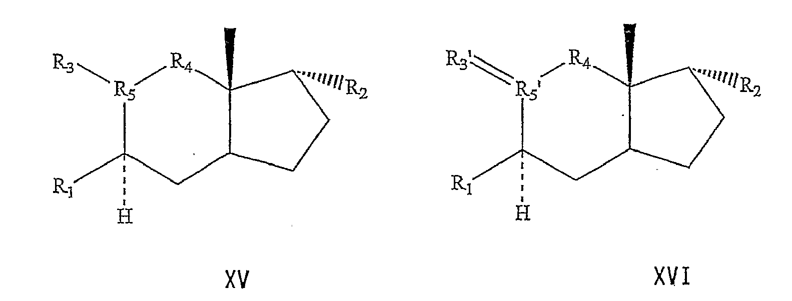 wherein R₁ is 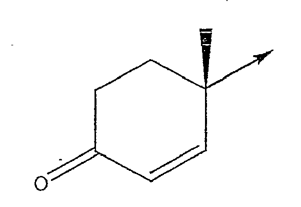 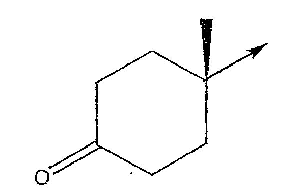 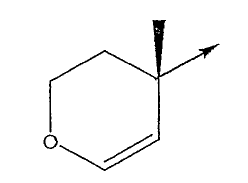 or 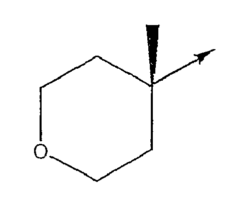 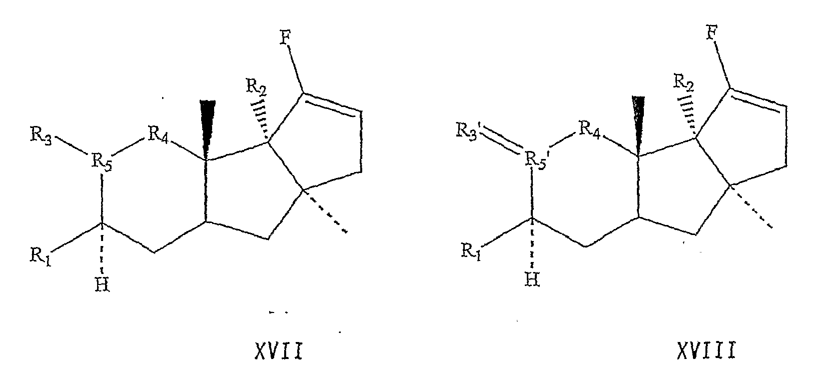 wherein R₁ is 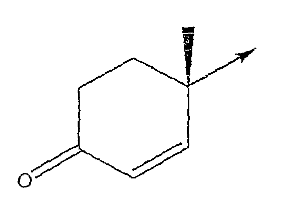 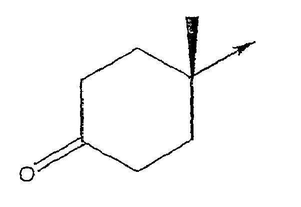 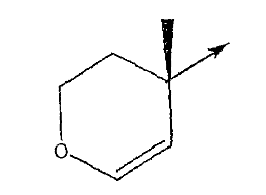 or 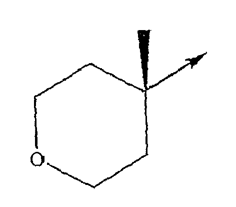 R₂ is H, OR, or halogen, R₃ is OR, SH, or NH₂, R₃' is O, S, or NH, R⁴ is O, S, NH, or CH₂, R₅ is N or CH₂, and R₅' is SO or CH₂.

Further suitable inhibitors are those adamantyl acetamides described in Patent Application WO 2004/056745. Thus, suitable inhibitors, in particular, are compounds of formula XIX: 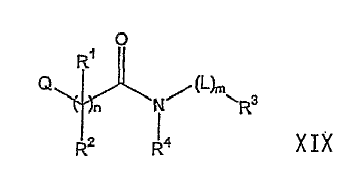 the *N*-oxide forms, the pharmaceutically acceptable addition salts and the stereochemically isomeric forms thereof, wherein
n represents an integer being O, 1 or 2;
m represents an integer being 0 or 1;
R¹ and R² each independently represents hydrogen, C₁₋₄alkyl, NR⁹R¹⁰, C₁₋₄alkyloxy, Het³-O-C₁₋₄alkyl; or
R¹ and R² taken together with the carbon atom with which they are attached form a carbonyl, or a C₃₋₆cycloalkyl; and where n is 2, either R¹ or R² may be absent to form an unsaturated bond;
R³ represents hydrogen, Ar¹, C₁₋₈alkyl, C₆₋₁₂cycloalkyl or a monovalent radical having one of the following formulae 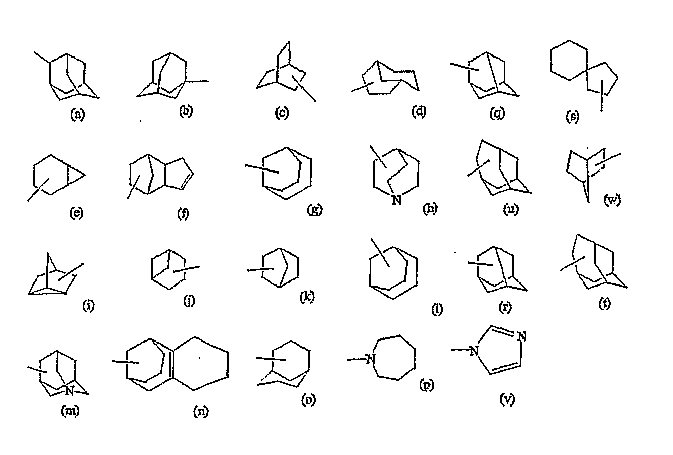 wherein said Ar¹, C₆₋₁₂cycloalkyl or monovalent radical may optionally be substituted with one, or where possible two or three substituents selected from the group consisting of C₁₋₄alkyl, C₁₋₄alkyloxy, phenyl, halo, oxo, carbonyl, 1,3-dioxolyl or hydroxy; in particular R³ represents a monovalent radical having formula a) or b) optionally substituted with one, or where possible two or three substituents selected from the group consisting of C₁₋₄alkyl, C₁₋₄alkyloxy, phenyl, halo, oxo, carbonyl, 1,3-dioxolyl or hydroxy;
R⁴ represents hydrogen, C₁₋₄alkyl, or C₂₋₄alkenyl; Q represents C₃₋₈cycloalkyl, Het¹ or Ar², wherein said C₃₋₈cycloalkyl, Het¹ or Ar² are optionally substituted with one or where possible more substituents selected from halo, C₁₋₄alkyl, C₁₋₄alkyloxy, hydroxy, nitro, Het⁴, phenyl, phenyloxy, C₁₋₄alkyloxycarbonyl, hydroxycarbonyl, NR⁵R⁶, C₁₋₄alkyloxy substituted with one or where possible two or three substituents each independently selected from C₁₋₄alkyl, hydroxycarbonyl, Het², C₁₋₄alkyl or NR⁷R⁸, C₂-₄alkenyl substituted with one substituent selected from phenyl-C₁₋₄alkyloxycarbonyl, C₁₋₄alkyloxycarbonyl, hydroxycarbonyl or Het⁵-carbonyl, and C₁₋₄alkyl substituted with one or where possible two or three substituents independently selected from halo, dimethylamine, trimethylamine, amine, cyano, Het⁶, Het⁷-carbonyl, C₁₋₄alkyloxycarbonyl or hydroxycarbonyl;
R⁵ and R⁶ are each independently selected from hydrogen, C₁₋₄alkyl, C₁₋₄alkyloxyC₁₋₄alkyl, C₁₋₄alkyloxycarbonyl, C₁₋₄alkylcarbonyl, C₁₋₄alkylcarbonyl substituted with one or where possible two or three substituents each independently selected from halo, C₁₋₄alkyl, and C₁₋₄alkyloxy or R⁵ and R⁶ each independently represent C₁₋₄alkyl substituted with phenyl;
R⁷ and R⁸ are each independently selected from hydrogen or C₁₋₄alkyl;
R⁹ and R¹⁰ are each independently selected from hydrogen, C₁₋₄alkyl or C₁₋₄alkyloxycarbonyl;
L represents C₁₋₄alkyl optionally substituted with one or where possible more substituents selected from C₁₋₄alkyl or phenyl;
- Het¹: represents a heterocycle selected from pyridinyl, piperinidyl, pyrimidinyl,pyrazinyl, piperazinyl, pyridazinyl, indolyl, isoindolyl, indolinyl, furanyl, benzofuranyl, thiazolyl, oxazolyl, isoxazolyl, isothiazolyl, benzothiophenyl, thiophenyl, 1,8-naphthyridinyl, 1,6-naphthyridinyl, quinolinyl 1,2,3,4-tetrahydro-quinolinyl, isoquinolinyl, 1,2,3,4-tetrahydro-isoquinolinyl, quinoxalinyl, quinazolinyl, phthalazinyl, 2H-benzopyranyl, 3,4-dihydro-2H-benzopyxanyl, 2H-benzothiopyranyl, 3,4-dihydro-2H-benzothiopyranyl or 1,3-benzodioxolyl;
- Het ²: represents a monocyclic heterocycle selected from piperidinyl, pyridinyl, pyridazinyl, pyrimidinyl, pyrazinyl, piperazinyl, 2H-pyrrolyl, pyrrolyl, 2-pyrrolinyl, 3-pyrrolinyl, pyrrolidinyl, or morpholinyl, said Het² optionally being substituted with one or where possible two or more substituents each independently selected from hydroxy, C₁₋₄alkyl or C₁₋₄alkyloxy;
- Het³: represents a monocyclic heterocycle selected from 2H-pyranyl, 4H-pyranyl, furanyl, tetrahydro-2H-pyranyl, pyridinyl, piperidinyl, or furanyl;
- Het⁴: represents a monocyclic heterocycle selected from pyridazinyl, pyrimidinyl, pyrrolidinyl, pyrazinyl, piperazinyl, triazolyl, tetrazolyl or morpholinyl, said Het⁴ optionally being substituted with one or where possible two or more substituents each idependently selected from hydroxy, carbonyl, C₁₋₄alkyl or C₁₋₄alkyloxy;
- Het⁵: represents a monocyclic heterocycle selected from pyridazinyl, pyrimidinyl, pyrrolidinyl, pyrazinyl, piperazinyl or morpholinyl, said Het⁵ optionally being substituted with one or where possible two or more substituents each independently selected from hydroxy, carbonyl, C₁₋₄alkyl or C₁₋₄alkyloxy; in particular piperazinyl or morpholinyl;
- Het⁶: represents a monocyclic heterocycle selected from pyridazinyl, pyrimidinyl, pyrrolidinyl, pyrazinyl, piperazinyl or morpholinyl, said Het⁶ optionally being substituted with one or where possible two or more substituents each independently selected from hydroxy, carbonyl, C₁₋₄alkyl or C₁₋₄alkyloxy;
- Het⁷: represents a monocyclic heterocycle selected from pyridazinyl, pyrimidinyl, pyrrolidinyl, pyrazinyl, piperazinyl or morpholinyl, said Het⁷ optionally being substituted with one or where possible two or more substituents each independently selected from hydroxy, carbonyl, C₁₋₄alkyl or C₁₋₄alkyloxy; in particular selected piperazinyl or morpholinyl;
- Ar¹: represents carbocyclic radicals containing one or more rings selected from the group consisting of phenyl, biphenyl, indenyl, 2,3-dihydroindenyl, fluorenyl, 5,6,7,8-tetrahydronaphtyl or naphthyl
- Ar²: represents carbocyclic radicals containing one or more rings selected from the group consisting of phenyl, biphenyl, benzocyclobutenyl, benzocycloheptanyl, benzosuberenyl, indenyl, 2,3-dihydroindenyl, fluorenyl, 1,2-dihydronaphthyl, 5,6,7,8-tetrahydronaphthyl or naphthyl.

Further suitable inhibitors are those amide derivatives described in Patent Application WO 2004/065351. Thus, suitable inhibitors, in particular, are compounds of formula XX: The present invention provides amide derivatives of the formula 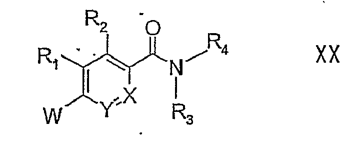 wherein
R₁ and R₂ are independently hydrogen, cyano, halo, nitro, trifluoromethyl, optionally substituted amino, alkyl, alkoxy, aryl, aralkyl, heteroaryl or heteroaralkyl; or
R₁ and R₂ combined together with the carbon atoms they are attached to form an optionally substituted 5- to 7-membered aromatic or heteroaromatic ring;
R₃ is optionally substituted lower alkyl; or
R₃ and R₂ combined together with the amide group to which R₃ is attached and the carbon atoms to which R₂ and the amide are attached form an optionally substituted 5- to 7-membered carbocyclic or heterocyclic ring;
R₄ is optionally substituted alkyl, cycloalkyl, heterocyclyl, aryl, aralkyl or heteroaralkyl; or
R₄ and R₃ taken together with the nitrogen atom to which they are attached form a 5- to 8-membered ring which may be optionally substituted or may contain another heteroatom selected from oxygen, nitrogen and sulfur; or
R₄ and R₃ taken together with the nitrogen atom to which they are attached form a 8- to 12-membered fused bicyclic ring, which may be optionally substituted or may contain another heteroatom selected from oxygen, nitrogen and sulfur;
W is -NR₅C(O)R₆, -NR₅C(O)OR₆, -NR₅C(O)NR₆R₇, -NR₅C(S)NR₆R₇, -NR₅S(O)₂R₆, -NR₅R₈, -C(O)NR₆R₇, -OR₉ or -OC(O)NR₆R₇ in which
R₅ and R₇ are independently hydrogen, optionally substituted alkyl or aralkyl; or
R₅ and R₁ are optionally substituted alkylene which combined together with the nitrogen atom to which R₅ is attached and the carbon atoms to which W and R₁ are attached form a 5- or 6-membered ring;
R₆ is optionally substituted alkyl, cycloalkyl, heterocyclyl, aryl, aralkyl or heteroaralkyl;
R₈ is optionally substituted alkyl, aralkyl or heteroaralkyl;
R₉ is hydrogen, optionally substituted alkyl, cycloalkyl, heterocyclyl, heterocyclo-allyl, aralkyl, heteroaralkyl, alkanoyl, aroyl or heteroaroyl; or W is aryl or heteroaryl; or W is hydrogen provided that R₁ is -NR₅Z in which Z is -C(O)R₆, -C(O)OR₆, -C(O)NR₆R₇, -C(S)NR₆R₇, -S(O)₂R₆, or -R₈; or W and R₁ combined together with the carbon atoms to which they are attached form a 6-membered aromatic or heteroaromatic ring optionally substituted with alkyl, alkoxy, aryl, heteroaryl, halo, -NR₅Z, -C(O)NR₆R₇, -OR₉ or -OC(O)NR₆R₇;
X and Y are independently CH or nitrogen; or
-X=Y- is -CH₂-, oxygen, sulfur or -NR₁₀- in which R₁₀ is hydrogen or lower alkyl; or a pharmaceutically acceptable, salt thereof.

Further suitable inhibitors are those of compounds of formulae XXI and XXII: 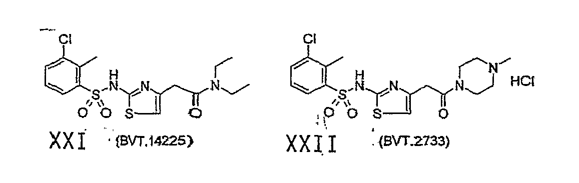 The 11-β-HSD-type 1 and/or type 2 inhibitors of the present invention can be utilized in the prevention and/or treatment of inflammation-induced and/or immune-mediated loss of bone and/or cartilage alone or in combination with at least one active ingredient being effective in the prevention and/or treatment of inflammation-induced and/or immune-mediated loss of bone and/or cartilage.

The drug products are produced by using an effective dose of the compounds of the invention or salts thereof, in addition to conventional adjuvants, carriers and additives. The dosage of the pharmaceutical agents may vary depending on the mode of administration, the age and weight of the patient, the nature and severity of the disorders to be treated and similar factors. The daily dose may be given as a single dose to be administered once a day, or divided into two or more daily doses, and is usually 5-100 mg/kg body weight, preferably 7-80 mg/kg body weight, more preferably 10-50 mg/kg body weight and most preferred 20 mg/kg body weight, related to a person weighing 70 kg.

Oral, sublingual, intravenous, intramuscular, intraarticular, intraarterial, intramedullar, intrathecal, intraventricular, intraocular, intracerebral, intracranial, respiratoral, intratracheal, nasopharhyngeal, transdermal, intradermal, subcutaneous, intraperitoneal, intranasal, enteral and/or topical administration and/or administration via rectal means, via infusion and/or via implant are suitable according to the invention. Oral administration of the compounds of the invention is particularly preferred. Galenical pharmaceutical presentations such as tablets, coated tablets, capsules, dispersible powders, granules, aqueous solutions, aqueous or oily substances, sirup, solutions or drops are used. Solid drug forms may comprise inert ingredients and carriers such as, for example, calcium carbonate, calcium phosphate, sodium phosphate, lactose, starch, mannitol, alginates, gelatin, guar gum, magnesium stearate or aluminium stearate, methylcellulose, talc, colloidal silicas, silicone oil, high molecular weight fatty acids (such as stearic acid), agar-agar or vegetable or animal fats and oils, solid high molecular weight polymers (such as polyethylene glycol); preparations suitable for oral administration may, if desired, comprise additional flavourings and/or sweetners. Liquid drug forms can be sterilized and/or, where appropriate, can comprise excipients such as preservatives, stabilizers, wetting agents, penetrants, emulsifiers, spreading agents, solubilizers, galts, sugars or sugar alcohols to control the osmotic pressu re or for buffering and/or viscosity regulators.

Examples of such additions are tartrate buffer and citrate buffer, ethanol, complexing agents (such as ethylenediaminetetraacetic acid and its non-toxic salts). Suitable for controling the viscosity are high molecular weight polymers such as, for example, liquid polyethylene oxide, microcrystalline celluloses, carboxymethylcelluloses, polyvinylpyrrolidones, dextrans or gelatin. Examples of solid carriers are starch, lactose, mannitol, methylcellulose, talc, colloidal silicas, higher molecular weight fatty acids (such as stearic acid), gelatin, agar-agar, calcium phosphate, magnesium stearate, animal and vegetable fats, solid high molecular weight polymers such as polyethylene glycol.

Oily suspensions for parenteral or topical uses may be vegetable, synthetic or semisynthetic oils such as, for example, liquid fatty acid esters with, in each case, 8 to 22 C atoms in the fatty acid chains, for example palmitic, lauric, tridecyclic, margaric, stearic, arachic, myristic, behenic, pentadecyclic, linoleic, elaidic, brasidic, erucic or oleic acid, which are esterified with monohydric to trihydric alcohols having 1 to 6 C atoms, such as, for example, methanol, ethanol, propanol, butanol, pentanol or iosmers thereof, glycol or glycerol. Examples of such fatty acid esters are commercially available miglyols, isopropyl myristate, isopropyl palmitate, isopropyl stearate, PEG 6-capric acid, caprylic/capric esters of saturated fatty alcohols, polyoxyethylene glycerol trioleates, ethyl oleate, waxy fatty acid esters such as artificial duch preen gland fat, coco fatty acid, isopropyl ester, oleyl oleate, decyl oleate, ethyl lactate, dibutyl phthalate, diisopropyl adipate, polyol fatty acid esters inter alia. Also suitable are silicone oils differing in viscosity or fatty alcohols such as isotridecyl alcohol, 2-octyldodecanol, cetylstearyl alcohol or oleyl alcohol, fatty acids such as, for example, oleic acid. It is also possible to use vegetable oils such as caster oil, almond oil, olive oil, sesame oil, cottonseed oil, peanut oil or soybean oil.

Suitable solvents, gel formers and solubilizers are water or water-miscible solvents. Suitable examples are alcohols such as, for example, ethanol or isopropyl alcohol, benzyl alcohol, 2-octyldodecanol, polyethylene glycols, phthalates, adipates, propylene gylcol, glycerol, di- or tripropylene gylcol, waxes, methyl Cellosolve, Cellosolve, esters, morpholines, dioxane, dimethyl sulfoxide, dimethylformamide, tetrahydrofuran, cyclohexanine, etc.

Film formers which can be used are cellulose ethers able to dissolve or swell both in water and in organic solvents such as, for example, hydroxypropylmethylcellulose, methylcellulose, ethylcellulose or soluble starches.

Combined forms of gel formers and film formers are also possible. In particular, ionic macromoelcules are used for this purpose, such as, for example, sodium carboxymethylcellulose, polyacrylic acid, polymethylacrylic acid and salts thereof, sodium amylopectin semiglycolate, alginic acid or propylene glycol alginate as sodium salt, gum arabic, xanthan gum, guar gum or carrageenan.

Further formulation aids which can be employed are glycerol, paraffin of differing viscosity, triethanolamine, collagen, allantoin, novantisolic acid.

It may also be necessary to use surfactants, emulsifiers or wetting agents for the formulation, such as, for example, Na lauryl sulfate, fatty alcohol ether sulfates, di-Na-N-lauryl-β-iminodipropionate, polyethoxylated castor oil or sorbitan monooelate, sorbitan monostearate, polysorbates (e.g. Tween), cetyl alcohol, lecithin, glyceryl monostearate, polyoxyethylene stearate, alkylphenol polyglycol ether, cetyltrimethylammonium chloride or mono/dialkylpolyglycol ether orthophosphoric acid monoethanolamine salts.

Stabilizers such as montmorillonites or colloidal silicas to stabilize emulsions or to prevent degradation of the active substances, such as antioxidants, for example tocopherals or butylated hydroxyanisole, or preservatives such as p-hydroxybenzoic esters, may likewise be necessary where appropriate to prepare the desired formulations.

Preparations for parenteral administration may be present in separate dose unit forms such as, for example, ampoules or vials. Solutions of the active ingredient are preferably used, preferably aqueous solutions and especially isotonic solutions, but also suspensions. These injection forms can be made available as a finished product or be prepared only immediately before use by mixing the active compound, e.g. the lyophilistate, where appropriate with further solid carriers, with the desired solvent or suspending agent.

Intranasal preparations may be in the form of aqueous or oily solutions or of aqueous or oily suspensions. They may also be in the form of lyophilistates which are prepared before use with the suitable solvent or suspending agent.

The manufacture, bottling and closure of the products takes place under the usual antimicrobial and aseptic conditions.

A further aspect of the invention encompasses a pharmaceutical composition comprising as an active ingredient an 11-β-HSD-type 1 and/or type 2 inhibitor or a salt thereof and a pharmaceutically acceptable carrier or diluent, wherein said 11-β-HSD-type 1 and/or type 2 inhibitor is selected from the group consisting of the formulas 1 bis 31 as defined above.

In a preferred embodiment, the pharmaceutical composition of the 11-β-HSD-type 1 and/or type 2 inhibitor has the structure of formula I as defined above.

In another preferred embodiment of the invention, the pharmaceutical composition is selected from the group consisting of the formula 13, 14, 24 and 25 as defined above.

In a further embodiment, the pharmaceutical composition preferably has the structure of formula II as defined above. More preferably, the structure of formula II is formula 16 as defined above.

In another embodiment of the present invention, the pharmaceutical composition has formula 7 as defined above.

According to the invention, a pharmaceutical composition is preferably for the prevention and/or treatment of inflammation-induced and/or immune-mediated loss of bone and/or cartilage, more preferably for the prevention and/or treatment of osteoporosis, postmenopausal osteoporosis, Paget's disease, lytic bone metastases, arthritis, osteoarthritis, rheumatoid arthritis, diseases, bone loss by cancer, bone loss by HIV, periodontitis, bone marrow inflammation, synovial inflammation, cartilage/bone erosion and/or proteoglycan damage.

The pharmaceutical composition of the present invention, in addition to an 11-β-HSD-type 1 and/or type 2 inhibitor and a pharmaceutically acceptable carrier or diluent, can comprise at least one active ingredient being effective in the prevention and/or treatment of inflammation-induced and/or immune-mediated loss of bone and/or cartilage.

The pharmaceutical compositions may be administered by any number of routes including, but not limited to oral, sublingual, intravenous, intramuscular, intraarticular, intraarterial, intramedullar, intrathecal, intraventricular, intraocular, intracerebral, intracranial, respiratoral, intratracheal, nasopharhyngeal, transdermal, intradermal, subcutaneous, intraperitoneal, intranasal, enteral and/or topical and/or via rectal means, via infusion and/or implant. Preferably, said route of administration is oral.

The term "pharmaceutically acceptable" means a non-toxid material that does not interfere with the effectiveness of the biological activity of the active ingredients. Such preparations may routinely contain pharmaceutically acceptable concentrations of salts, buffering agents, preservatives, compatible carriers, supplementary immune potentiating agents such as adjuvants and cytokines and optionally other therapeutic agents such as chemotherapeutic agents.

When used in medicine, the salts should be pharmaceutically accceptable, but non-pharmaceutically acceptable salts may conveniently be used to prepare pharmaceutically acceptable salts thereof and are not excluded from the scope of the invention.

The pharmaceutical compositions may contain suitable buffering agents, including acetic acid in a salt; citric acid in a salt; boric acid in a salt; and phosphoric acid in a salt.

The pharmaceutical compositons optionally may also contain suitable preservatives such as benzalkonium chloride, chlorobutanol, parabenes and thiomersal.

The pharmaceutical compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier or both, and then, if necessary, shaping the product.

Compositions suitable for oral administration may be presented as discrete units such as capsules, tablets, lozenges, each containing a predetermined amount of the active compound. Other compounds include suspensions in aqueous liquids or non-aqueous liquids such as sirup, elixir or an emulsion.

Compositions suitable for parenteral administration conveniently comprise a sterile aqueous or non-aqueous preparation which is preferably isotonic with the blood of the recipient. This preparation may be formulated according to known methods using suitable dispersing or wetting agents and suspending agents. The sterile injectable preparation also may be a sterile injectable solution or suspension in a non-toxic parenterally acceptable diluent or solvent, for example, as a solution in 1,3-butane, diol. Among the acceptable vehicles and solvents that may be employed are water, Ringer's solution, and isotonic sodium chloride solution. In addition, sterile fixed oils are conventionally employed as a solvent or suspending medium. For this purpose, any bland fixed oil may be employed including synthetic mono- or diglycerides. In addition, fatty acids such as oleic acid may be used in the preparation of injectables.

Carrier formulations suitable for oral, subcutaneous, intravenous, intramuscular etc. administrations can be found in Remington's Pharmaceutical Sciences, Mack Publishing Co., Easton, PA.

In a preferred embodiment of the invention, the pharmaceutical compositions are administered to a mammal, preferably a human, in a dose of 5-100 mg/kg body weight per day, more preferably 7-80 mg/kg body weight per day, still more preferably 10-50 mg/kg body weight per day and most preferably 20 mg/kg body weight per day. This dose refers to a person weighing 70 kg.

In another preferred embodiment of the invention, the pharmaceutical composition is for the inhibition of osteoclast activity, since imbalances between osteclast and osteoblast activities toward the osteclast activities results in skeletal abnormalities characterized by loss of bone and/or cartilage.

### Examples

### Example 1

### Adjuvant-induced arthritis (AIA)

An intradermal injection, at the base of the tail, with heat killed Mycobaterium tuberculosum in incomplete Feund's Adjuvans results in destructive arthritis within 14 days in susceptible DA or LEW inbred rat strains. AIA can also be induced with cell walls from other bacterial types in IFA, although the arthritogenicity varies. Increased synthesis of tumor necrosis factor a (TNF-a), inter-leukin 1 (IL-1) and IL-6 is detected as early as day four after adjuvant injection. The disease progresses rapidly over several weeks in what appears clinically to be a monophasic process. Granulocytes and autoreactive CD41 cells play major roles in the disease. Humoral immune mechanisms appear not to contribute to the disease process. This unique rat disease model represents a systemic process that involves not only the joints but also the gastrointestinal and genitourinary tracts, the skin and the eyes. Although AIA clinically and histologically has similarities to human rheumatoid arthritis.

In this animal model it has impressively been demonstrated that bone loss and partially the related cartilage destruction essentially depends on the activation of osteoclasts by T-cells. Therefore this animal model ideally serves to investigate mechanisms and targets that might be suitable for the develop ment of novel therapeutics with improved therapeutic efficacy. In fact, most current treatments for arthritis and other conditions associated with immune mediated bone loss only ameliorate inflammation but fail to halt bone and cartilage loss.

Figure 2 shows the effect of 18-β-glycyrrhetinic acid (BX-1) on inflammation, as well as bone and cartilage loss. BX-1 early: BX-1 injected i.d. at the time of disease induction (day0) and day2, day4 BX-1 late: BX-1 injected i.d. at first signs of arthritis, day9, day11, day13 Samples are from both left and right hind limb of three rats per group of a representative experiment Data are shown as SEM

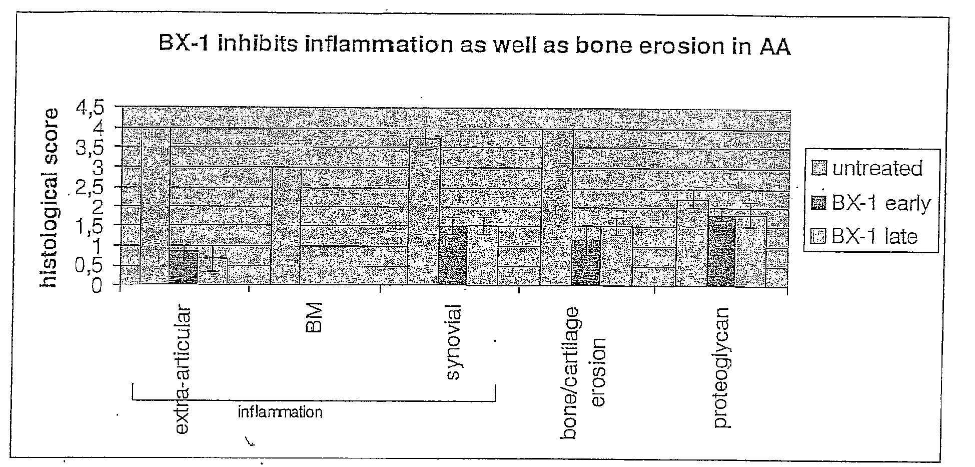

### Histology

Excised rat joints were stained with H&E. A synovial histology score was determined on the stained sections using a semiquantitative scale that measures synovial inflammation (0-4), bone and cartilage erosions (0-4), marrow infiltration (0-4), and extra-particular inflammation (0-4) (maximum score, 16).

### Statistics

Two-tailed unpaired Student ttests were used to compare Ab levels, cytokine levels, clinical arthritis scores, and histology scores using StatView (SAS Institute, Cary, NC) and Mathsoft computer software (Mathsoft, Cambridge, MA).

### Histological results of hind joint sections from arthritic rats

Rat ankle slides were histologically evaluated according to five criteria (blind evaluation by DL Boyle et al., University of California in San Diego, (J. Immunol., Jan 2002; 168: 51 - 56.):
1. Extra-articular inflammation
2. Bone marrow inflammation (BM)
3. Synovial inflammation
4. Gartilage/bone erosion
5. Proteoglycan damage

The complete lack of infiltration of the bone marrow has not been observed with any short term and/or discontinued treatment with a small molecule drug before.

The data further indicate that BX-1 (18-β-glycyrrhetinic acid) positively influence all arms of the pathology of arthritis; T-cell and dendritic cell activation, systemic inflammation, and bone marrow infiltration. Similar effects were seen with the hemisuccinate of BX-1, carbenoxolone (not shown).

The histological findings might explain why the animals go in remission upon late treatment, i.e. after the onset of disease and why there is absolutely no sign of re-exacerbation of disease after cessation of treatment in any model we have investigated so far; i.e. adjuvant arthritis and pristane-induced arthritis (not shown).

Over all, these data suggest that BX-1 may be an ideal drug to reduce inflammation-induced and/or immune bone destruction as observed not only in rheumatoid arthritis, but also periodontal diseases and other inflammatory conditions. In fact, the pathology of periodontal diesase and other pathologies resulting in bone destruction appears to follow a similar pathway as this is currently accepted for bone destruction in rheumatoid arthritis (Annu. Rev. Immunol., Jan 2002; 20: 795 - 823), which opens new, *ad hoc* opportunities for BX-1 and related drugs. Since BX-1 is an established inhibitor of 11-β-HSD type 1 and type 2, enzymes blocking these with inhibitors appears a most promising avenue to cure diseases associated with inflammation and/or immune mediated bone loss.

### Example 2

### Materials

Cell culture reagents were purchased from Invitrogen (Carlsbad, CA), [1,2,6,7-³H]-cortisone from American Radiolabeled Chemicals (St. Louis, MO) and [1,2,6,7-³H]-cortisol from Amersham Biosciences (General Electrics Healthcare, Piscataway, NJ).
Thin layer chromatography (TLC) plates (SIL G-25 UV254) were purchased from Macherey-Nagel, Oensingen, Switzerland.

### Assay for 11β-HSD activity

The screening assay used to determine inhibition of 11β-HSD enzyme activity is based on the conversion of radiolabelled cortisone or cortisol in cell lysates from HEK-293 cells, stably transfected with either human 11β-HSD1 or human 11β-HSD2 (Schweizer et al. 2003, Frick et a. 2004). Cells were grown in 10 cm dishes to 80% confluence and incubated for 16 h in steroid-free medium (charcoal-treated fetal calf serum (FCS) from HyClone, Logan, Utah). Cells were rinsed once with phosphate-buffered saline (PBS), dettached and centrifuged for 3 min at 150 x g. The supernatant was removed and the cell pellet quick-frozen in a dry-ice ethanol bath. At the day of experiment, cell pellets were resuspended in buffer TS2 (100 mM NaCl, 1 mM EGTA, 1 mM EDTA, 1 mM MgCl₂, 250 mM sucrose, 20 mM Tris-HCl, pH 7.4), sonicated and activities determined immediately. The rate of conversion of cortisol to cortisone or the reverse reaction was determined in 96-well optical PCR reaction plates (Applied Bio systems, Foster City, CA) in a final volume of 22 µl, and the tubes were capped during the reaction to avoid evaporation.

### Determination of oxidase activity:

Reactions were initiated by simultaneously adding 10 µl of cell lysate and 12 µl of TS2 buffer containing the appropriate concentration of the inhibitory compound to be tested, NAD⁺, 30 nCi of [1,2,6,7-³H]-cortisol and unlabeled cortisol. A final concentration of 400 µM NAD⁺ and 25 nM cortisol were used. Stock solutions of the inhibitors in methanol or DMSO were diluted in TS2 buffer to yield the appropriate concentrations, whereby the concentration of methanol or DMSO in the reactions were kept below 0.1%.
Control reactions with or without 0.1% of the solvent were performed. Incubation was at 37°C for 10 min with shaking, reactions were terminated by adding 10 µl of stop solution containing 2 mM of unlabeled cortisol and cortisone dissolved in methanol. The conversion of radiolabeled cortisol was determined by separation of cortisol and cortisone using TLC and a solvent system of 9:1 (v/v) chloroform:methanol, followed by scintillation counting. In absence of inhibitors approximately 30% of cortisol was converted to cortisone.

### Determination of reductase activity:

Reactions were initiated simultaneously by adding 10 µl of cell lysate and 12 µl of TS2 buffer containing the appropriate concentration of the inhibitory compound to be tested, NADPH, 30 nCi of [1,2,6,7-³H]-cortisone and unlabeled cortisone, whereby final concentrations were 400 µM NADPH and 100 nM cortisone. Activities were determined immediately after cell disruption by measuring the conversion of radiolabeled cortisone to cortisol for 10 min.
Enzyme kinetics were analyzed by non-linear regression using Data Analysis Toolbox (MDL Information Systems Inc.) assuming first-order rate kinetics. Data represent mean ± SD of four to five independent experiments.

### References

Schweizer, R. A., Atanasov, A. G., Frey, B. M., and Odermatt, A. (2003) Mol Cell Endocrinol 212, 41-49. Christoph Frick, Atanas G. Atanasov, Peter Arnold, Juris Ozols, and Alex Odermatt (2004) J Biol Chem, 279, 131-138.

### Example 3

Inhibition of 11β-HSD1 was determined at 100 nM cortisone, inhibtion of 11β-HSD2 at 25 nM cortis as substrates (at approximately 30% of apparent Km concentrations).

Assay with 20 µM of the corresponding compound in the reaction mixture, added simultaneously wit the substrate:

| | | | | |
|---|---|---|---|---|
| 11β-HSD1 | 11β-HSD1 | % of control | 11β-HSD2 | % of control |
| control | | 99.9999986 | | 100 |
| 10 µM CBX | | 4.43030125 | | 15.52151455 |
| BNW1 | | 102.112595 | | 96.77455646 |
| BNW2 | | 78.8440316 | | 77.95067459 |
| BNW3 | | 60.2536577 | | 53.56660046 |
| BNW4 | | 82.2425505 | | 95.04764105 |
| BNW5 | | 69.7522595 | | 97.47129918 |
| BNW6 | | 79.6439869 | | 145.0319346 |
| BNW7 | | 9.59257261 * | | 139.5062669 |
| BNW8 | | 41.7056688 | | 102.7042587 |
| BNW9 | | 30.6544131 | | 77.43471825 |
| BNW10 | | 64.325535 | | 128.6701314 |
| BNW11 | | 70.0994104 | | 120.918247 |
| BNW12 | | 85.3624514 | | 132.1217751 |
| BNW13 | | 3.87940281 * | | 14.37405632 * |
| BNW14 | | 20.1589034 * | | 25.52077188 * |
| BNW15 | | 50.3669741 | | 56.94887208 |
| BNW16 | | 2.70799056* | | 27.37171929 |
| BNW17 | | 88.2225144 | | 120.1411745 |
| BNW18 | | 92.0338994 | | 82.80931996 |
| BNW19 | | 51.0824709 | | 73.62927124 |
| BNW20 | | 46.8261929 | | 120.655235 |
| BNW21 | | 48.9418364 | | 121.5916615 |
| BNW22 | | 41.3182359 | | 104.3264654 |
| BNW23 | | 85.0676295 | | 132.6608 |
| BNW24 | | 3.93928545 * | | 13.34505396 * |
| BNW25 | | 2.88437681* | | 13.92786069 * |
| BNW26 | | 94.0659079 | | 136.7564992 |
| BNW27 | | 78.6422701 | | 126.3527217 |
| BNW28 | | 76.7298316 | | 136.975487 |
| BNW29 | | 75.2887485 | | 115.4231371 |
| BNW30 | | 48.3569192 | | 139.9742227 |

### Example 4

**Determination of IC50 values, using 7 different inhibitor concentrations at factor 2 intervalls:**

| | | | | | | | |
|---|---|---|---|---|---|---|---|
| 11β-HSD1 | **all values** in **µM** | | | | | | |
| | | **BNW 7** | **BNW 13** | **BNW 14** | **BNW 16** | **BNW 24** | **BNW 25** |
| IC 50 1 | 1 | 1.95e+0 | 6.66e-1 | 2.75e+0 | 1.49e-1 | 7.33e-1 | 1.47e-1 |
| | 2 | 1.91e+0 | 7.56e-1 | 3.09e+0 | 1.68e-1 | 9.05e-1 | 2.06e-1 |
| | 3 | 2.24e+0 | 6.52e-1 | 2.58e+0 | 1.14e-1 | 7.74e-1 | 1.61e-1 |
| | Mittelwert | 2.03e+0 | 6.91e-1 | 2.81e+0 | 1.44e-1 | 8.04e-1 | 1.72e-1 |
| 11β-HSD2 | Standardabweichung | 0.178522195 | 0.05642599 | 0.25800854 | 0.02724464 | 0.08980411 | 0.03079395 |
| IC 50 | 1 | did not inhibit | out of range | out of range | out of range | out of range | out of range |
| | 2 | did not inhibit | 2.63e-1 | 2.01e+0 | 4.04e+0 | 1.69e-1 | 5.46e-2 |
| | 3 | did not inhibit | 2.99e-1 | 2.69e+0 | 3.87e+0 | 2.34e-1 | 6.49e-2 |
| | Mittelwert n.d. | n.d. | 2.81e-1 | 2.35e+0 | 3.95e+0 | 2.02e-1 | 5.97e-2 |
| | Standardabweichung | n.d. | 0.02520514 | 0.48148793 | 0.11686909 | 0.04659304 | 0.00731635 |

## Claims

1. Implant for the prevention or treatment of inflammation-induced and/or immune mediated loss of bone and/or cartilage comprising an 11-β-HSD-type 1 and type 2 inhibitor, or a pharmaceutically acceptable salt thereof.

2. Pharmaceutical composition comprising a preparation of an 11-β-HSD-type 1 and type 2 inhibitor, or a pharmaceutically acceptable salt thereof, for oral use, and a preparation of an 11-β-HSD-type 1 and type 2 inhibitor, or a pharmaceutically acceptable salt thereof, for topical use and/or use as an implant.

3. Pharmaceutical composition or implant according to any one of claims 1 or 2, wherein any of the 11-β-HSD-type 1 and type 2 inhibitor is 18-β-glycyrrhetinic acid or a derivative thereof such as glycyrrhizin or carbenoxolone.

4. Pharmaceutical composition for preventing or treating inflammation-induced and/or immune mediated loss of bone and/or cartilage comprising a preparation of an 11-β-HSD-type 1 and type 2 inhibitor, or a pharmaceutically acceptable salt thereof, and an antimicrobial agent.

5. Pharmaceutical composition or implant according to any one of claims 1 to 4, wherein the inflammation-induced and/or immune mediated loss of bone and/or cartilage is associated with periodontitis and/or rheumatoid arthritis.

6. Pharmaceutical composition according to any one of claims 1 to 5, wherein the 11-β-HSD-type 1 and type 2 inhibitor, or a pharmaceutically acceptable salt thereof, is selected from the group consisting of
| Compound Name | Structure |
|---|---|
| Formula 1 | 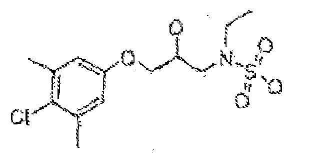 |
| Formula 2 | 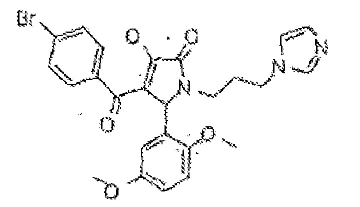 |
| Formula 3 | 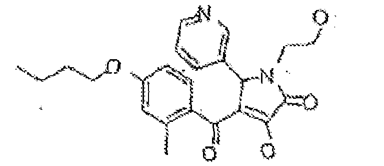 |
| Formula 4 | 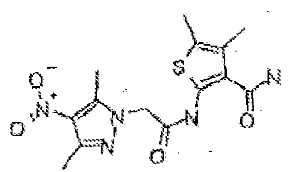 |
| Formula 5 | 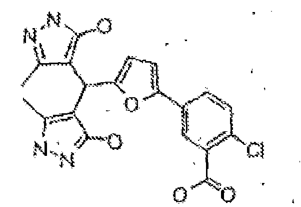 |
| Formula 6 | 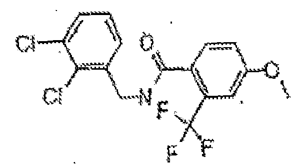 |
| Formula 7 | 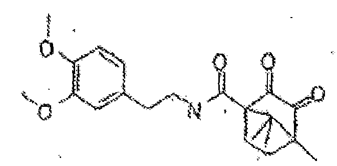 |
| Formula 8 | 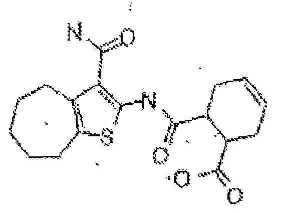 |
| Formula 9 | 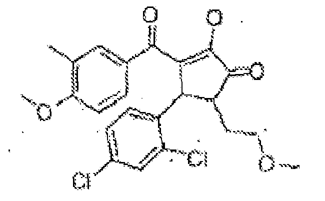 |
| Formula 10 | 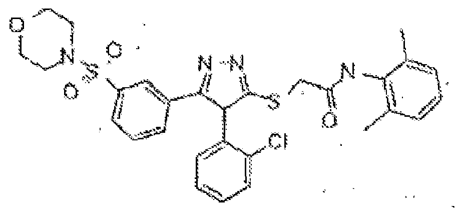 |
| Formula 11 | 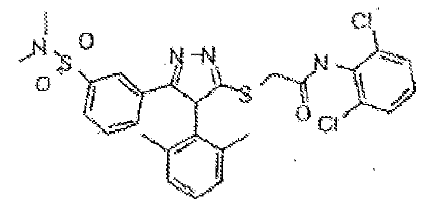 |
| Formula 12 | 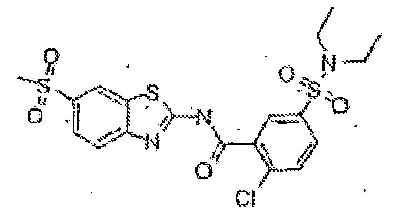 |
| Formula 13 | 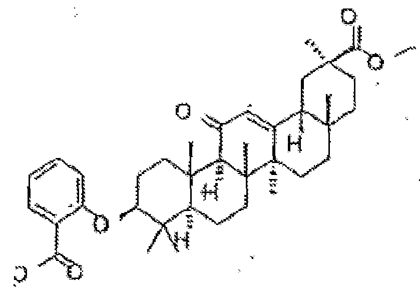 |
| Formula 15 | 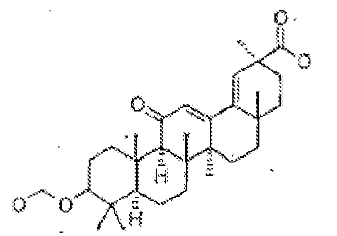 |
| Formula 16 | 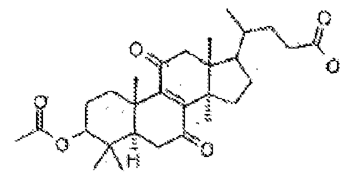 |
| Formula 17 | 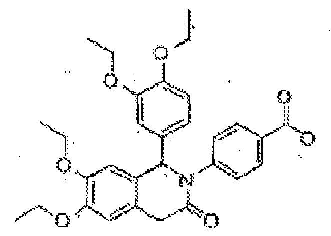 |
| Formula 18 | 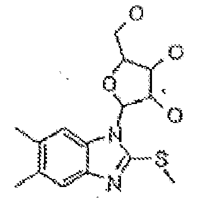 |
| Formula 19 | 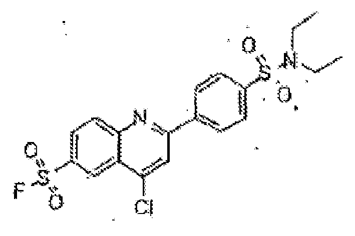 |
| Formula 20 | 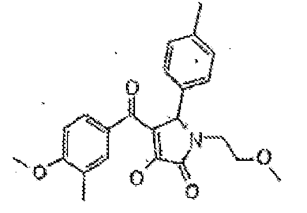 |
| Formula 21 | 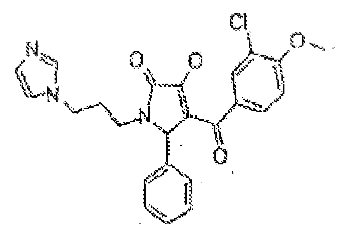 |
| Formula 22 | 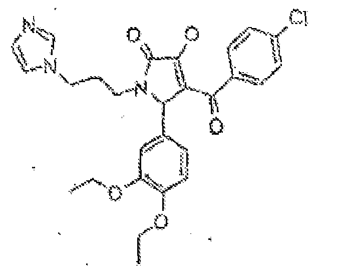 |
| Formula 23 | 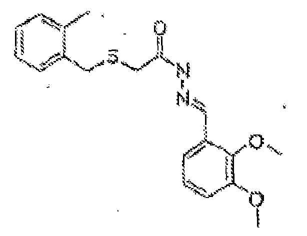 |
| Formula 25 | 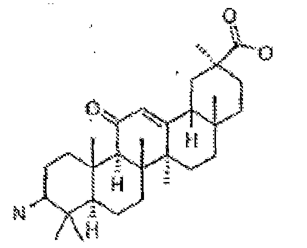 |
| Formula 26 | 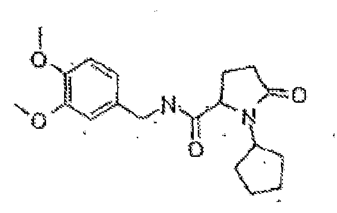 |
| Formula 27 | 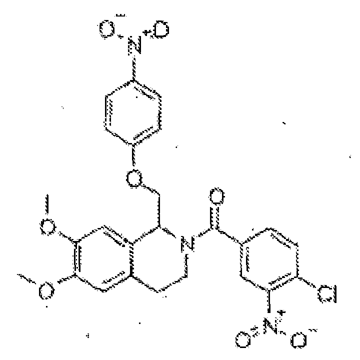 |
| Formula 28 | 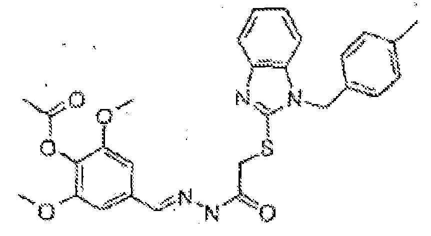 |
| Formula 29 | 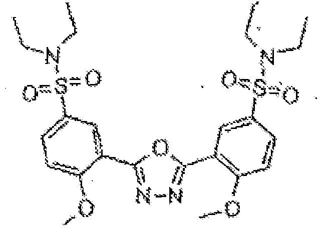 |
| Formula 30 | 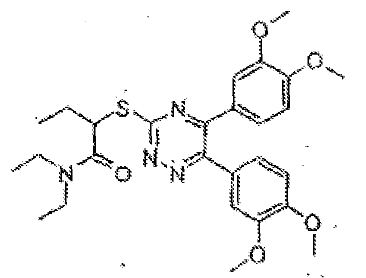 |
| Formula 31 | 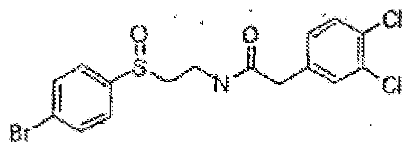 |
